# EUROPEAN PATENT SPECIFICATION

(11) **EP 1 551 868 B1**
(45) Date of publication and mention of the grant of the patent: **07.01.2009**
(21) Application number: 03769423.9
(22) Date of filing: 20.10.2003
(51) Int. Cl.: C07K 14/00

(54) **FACTOR INVOLVED IN METASTASIS AND USES THEREOF**
AN DER METASTASE BETEILIGTER FAKTOR UND VERWENDUNGEN DAFÜR
FACTEUR IMPLIQUE DANS LA METASTASE ET SES UTILISATIONS

(30) Priority: 18.10.2002 EP 02023384
(43) Date of publication of application: 13.07.2005
(73) Proprietor: Silence Therapeutics AG, 13125 Berlin (DE)
(72) Inventor: KLIPPEL-GIESE, Anke, 10779 Berlin (DE); KAUFMANN, Jörg, 13437 Berlin (DE); SCHWARZER, Rolf, 13187 Berlin (DE)
(74) Representative: Bohmann, Armin K.
(86) International application number: PCT/EP2003/011604
(87) International publication number: WO 2004/035615

(56) References cited:
- WO-A-00/77022
- WO-A-02/46465
- SHOSHANI TZIPORA ET AL: "Identification of a novel hypoxia-inducible factor 1-responsive gene, RTP801, involved in apoptosis." MOLECULAR AND CELLULAR BIOLOGY. UNITED STATES APR 2002, vol. 22, no. 7, April 2002 (2002-04), pages 2283-2293, XP002267737 ISSN: 0270-7306
- SEMENZA ET AL ADV.EXP.MED.BIOL. vol. 475, 2000, pages 123 - 130, XP008068313

### SEQUENCE LISTING

<110> atugen AG
<120> New factor for metastasis and uses thereof
<130> A 19011 PCT
<160> 17
<170> PatentIn version 3.1
<210> 1
   <211> 232
   <212> PRT
   <213> Homo sapiens
<220>
   <221> MISC_FEATURE
   <223> amino acid sequence of NMF
<400> 1
   Met Pro Ser Leu Trp Asp Arg Phe Ser Ser Ser Ser Thr Ser Ser Ser
   1               5                   10                  15
   Pro Ser Ser Leu Pro Arg Thr Pro Thr Pro Asp Arg Pro Pro Arg Ser
               20                  25                  30
   Ala Trp Gly Ser Ala Thr Arg Glu Glu Gly Phe Asp Arg Ser Thr Ser
           35                  40                  45
   Leu Glu Ser Ser Asp Cys Glu Ser Leu Asp Ser Ser Asn Ser Gly Phe
       50                  55                  60
   Gly Pro Glu Glu Asp Thr Ala Tyr Leu Asp Gly Val Ser Leu Pro Asp
   65                  70                  75                  80
   Phe Glu Leu Leu Ser Asp Pro Glu Asp Glu His Leu Cys Ala Asn Leu
                   85                  90                  95
   Met Gln Leu Leu Gln Glu Ser Leu Ala Gln Ala Arg Leu Gly Ser Arg
               100                 105                 110
   Arg Pro Ala Arg Leu Leu Met Pro Ser Gln Leu Val Ser Gln Val Gly
           115                 120                 125
   Lys Glu Leu Leu Arg Leu Ala Tyr Ser Glu Pro Cys Gly Leu Arg Gly
       130                 135                 140
   Ala Leu Leu Asp Val Cys Val Glu Gln Gly Lys Ser Cys His Ser Val
   145                 150                 155                 160
   Gly Gln Leu Ala Leu Asp Pro Ser Leu Val Pro Thr Phe Gln Leu Thr
   165 170 175
   Leu Val Leu Arg Leu Asp Ser Arg Leu Trp Pro Lys Ile Gln Gly Leu
   180 185 190
   Phe Ser Ser Ala Asn Ser Pro Phe Leu Pro Gly Phe Ser Gln Ser Leu
   195 200 205
   Thr Leu Ser Thr Gly Phe Arg Val Ile Lys Lys Lys Leu Tyr Ser Ser
   210 215 220
   Glu Gln Leu Leu Ile Glu Glu Cys
   225 230
<210> 2
   <211> 1760
   <212> DNA
   <213> Homo sapiens
<400> 2
   gcagcaggcc aagggggagg tgcgagcgtg gacctgggac gggtctgggc ggctctcggt 60
   ggttggcacg ggttcgcaca cccattcaag cggcaggacg cacttgtctt agcagttctc 120
   gctgaccgcg ctagctgcgg cttctacgct ccggcactct gagttcatca gcaaacgccc 180
   tggcgtctgt cctcaccatg cctagccttt gggaccgctt ctcgtcgtcg tccacctcct 240
   cttcgccctc gtccttgccc cgaactccca ccccagatcg gccgccgcgc tcagcctggg 300
   ggtcggcgac ccgggaggag gggtttgacc gctccacgag cctggagagc tcggactgcg 360
   agtccctgga cagcagcaac agtggcttcg ggccggagga agacacggct tacctggatg 420
   gggtgtcgtt gcccgacttc gagctgctca gtgaccctga ggatgaacac ttgtgtgcca 480
   acctgatgca gctgctgcag gagagcctgg cccaggcgcg gctgggctct cgacgccctg 540
   cgcgcctgct gatgcctagc cagttggtaa gccaggtggg caaagaacta ctgcgcctgg 600
   cctacagcga gccgtgcggc ctgcgggggg cgctgctgga cgtctgcgtg gagcagggca 660
   agagctgcca cagcgtgggc cagctggcac tcgaccccag cctggtgccc accttccagc 720
   tgaccctcgt gctgcgcctg gactcacgac tctggcccaa gatccagggg ctgtttagct 780
   ccgccaactc tcccttcctc cctggcttca gccagtccct gacgctgagc actggcttcc 840
   gagtcatcaa gaagaagctg tacagctcgg aacagctgct cattgaggag tgttgaactt 900
   caacctgagg gggccgacag tgccctccaa gacagagacg actgaacttt tggggtggag 960
   actagaggca ggagctgagg gactgattcc agtggttgga aaactgaggc agccacctaa 1020
   ggtggaggtg ggggaatagt gtttcccagg aagctcattg agttgtgtgc gggtggctgt 1080
   gcattgggga cacatacccc tcagtactgt agcatggaac aaaggcttag gggccaacaa 1140
   ggcttccagc tggatgtgtg tgtagcatgt accttattat ttttgttact gacagttaac 1200
   agtggtgtga catccagaga gcagctgggc tgctcccgcc ccagcctggc ccagggtgaa 1260
   ggaagaggca cgtgctcctc agagcagccg gagggagggg ggaggtcgga ggtcgtggag 1320
   gtggtttgtg tatcttactg gtctgaaggg accaagtgtg tttgttgttt gttttgtatc 1380
   ttgtttttct gatcggagca tcactactga cctgttgtag gcagctatct tacagacgca 1440
   tgaatgtaag agtaggaagg ggtgggtgtc agggatcact tgggatcttt gacacttgaa 1500
   aaattacacc tggcagctgc gtttaagcct tcccccatcg tgtactgcag agttgagctg 1560
   gcaggggagg ggctgagagg gtgggggctg gaacccctcc ccgggaggag tgccatctgg 1620
   gtcttccatc tagaactgtt tacatgaaga taagatactc actgttcatg aatacacttg 1680
   atgttcaagt attaagacct atgcaatatt ttttactttt ctaataaaca tgtttgttaa 1740
   aacaaaaaaa aaaaaaaaaa 1760
<210> 3
   <211> 699
   <212> DNA
   <213> Homo sapiens
<400> 3
   atgcctagcc tttgggaccg cttctcgtcg tcgtccacct cctcttcgcc ctcgtccttg 60
   ccccgaactc ccaccccaga tcggccgccg cgctcagcct gggggtcggc gacccgggag 120
   gaggggtttg accgctccac gagcctggag agctcggact gcgagtccct ggacagcagc 180
   aacagtggct tcgggccgga ggaagacacg gcttacctgg atggggtgtc gttgcccgac 240
   ttcgagctgc tcagtgaccc tgaggatgaa cacttgtgtg ccaacctgat gcagctgctg 300
   caggagagcc tggcccaggc gcggctgggc tctcgacgcc ctgcgcgcct gctgatgcct 360
   agccagttgg taagccaggt gggcaaagaa ctactgcgcc tggcctacag cgagccgtgc 420
   ggcctgcggg gggcgctgct ggacgtctgc gtggagcagg gcaagagctg ccacagcgtg 480
   ggccagctgg cactcgaccc cagcctggtg cccaccttcc agctgaccct cgtgctgcgc 540
   ctggactcac gactctggcc caagatccag gggctgttta gctccgccaa ctctcccttc 600
   ctccctggct tcagccagtc cctgacgctg agcactggct tccgagtcat caagaagaag 660
   ctgtacagct cggaacagct gctcattgag gagtgttga 699
<210> 4
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 4
   gcucaactct gcagtacacg a 21
<210> 5
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 5
   cuugguccct tcagaccagu a 21
<210> 6
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 6
   caguuutcca accacuggaa u 21
<210> 7
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 7
   cccaaaagtt cagtcgucuc u 21
<210> 8
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 8
   gcuccugcct ctagtcucca c 21
<210> 9
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 9
   guguucatcc tcagggucau c 21
<210> 10
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 10
   ggucagtagt gatgcuccga u 21
<210> 11
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 11
   cuuaccaact ggctaggcau c 21
<210> 12
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 12
   ccgaaaagaa cagtgcucuc u 21
<210> 13
   <211> 21
   <212> DNA
   <213> Artificial Sequence
<220>
   <223> antisense oligonucleotide
<220>
   <221> misc_feature
   <222> (1)..(6)
   <223> RNA
<220>
   <221> misc_feature
   <222> (7)..(15)
   <223> DNA linked through phosphorothioate linkages
<220>
   <221> misc_feature
   <222> (16)..(21)
   <223> RNA
<400> 13
   gcucguccct gtagtgucca c 21
<210> 14
   <211> 54
   <212> RNA
   <213> Artificial Sequence
<220>
   <223> siRNA
<400> 14
   gggaaugaac cacuggaaua gcaaaaaaaa aaaagcuucc agugguucau uccc 54
<210> 15
   <211> 54
   <212> RNA
   <213> Artificial Sequence
<220>
   <223> siRNA
<400> 15
   acugagcaag aggcuuugga gaaaaaaaaa aaacuccaaa gccucuugcu cagu 54
<210> 16
   <211> 54
   <212> RNA
   <213> Artificial Sequence
<220>
   <223> siRNA
<400> 16
   guggagacua gaggcaggag caaaaaaaaa aaagcuccug ccucuagucu ccac 54
<210> 17
   <211> 187
   <212> DNA
   <213> Artificial
<400> 17
   gaattcctat ttcccatgat tccttcatat ttgcatattt ttaaaatgga ctatcatatg     60
   cttaccgtaa cttgaaagta tttcgatttc ttggctttat atatcttggg aaaggacgaa    120
   acaccgggag actagaggca ggagcaaaaa aaaaaactcc tgcctctagt ctccactttt    180
   tctcgag                                                              187

## Description

The present invention is related to the use of a polypeptide factor, and a nucleic acid coding therefore, as a downstream target, as a marker for PI 3-kinase regulated process and in the generation and manufacture of a medicament and a therapeutic agent.

Modern drug development no longer relies on a more or less heuristic approach but typically involves the elucidation of the molecular mechanism underlying a disease or a condition, the identification of candidate target molecules and the evaluation of said target molecules. It is obvious that the identification of a candidate target molecule is essential to such process. With the sequencing of the human genome and publishing of respective sequence data, in principle, all of the coding nucleic acids of man are available. However, a serious limitation to this data is that typically no annotation of the function of said sequence is given. Still, the mere knowledge of a coding nucleic acid sequence is not sufficient to predict the polypeptide's function *in vivo*. Accordingly, from studying the databank entries without any properly validated annotation the one skilled in the art cannot get any technical teaching on how to use this nucleic acid data. *In silico* methods allow a first possible annotation which, however, is prone to errors and does not necessarily reflect the real function of a polypeptide encoded by a respective nucleic acid sequences. Putting said polypeptide in the proper *in vivo* and *in situ* context and elucidating its function there, is still a demanding task which gives rise to surprising findings.

Once a validated target molecule, which is herein referred to also as target or target molecule, is available, drug candidates directed thereto may be screened or developed and subsequently tested. In many cases such drug candidates are members of a compound library which may consist of synthetic or natural compounds. Also the use of combinatorial libraries is common. Such compound libraries are herein also referred to as candidate compound libraries. Although in the past this approach has proven to be successful, it is still time and money consuming.

Still, numerous tumours and cancers are a big threat to human health. In order to create safer and more powerful drugs having less side effects, it is necessary to know about target molecules which, upon being addressed by appropriate compounds, may specifically and selectively be influenced in their activity or presence. Because of the preferably selective and specific interaction between the compound, which may be a potential or candidate drug, and the target, the target's function in a disease or diseased condition such as, for example, cancer, tumorigenesis and metastasis, may be influenced and thus the disease treated or prevented and the diseased condition ameliorated, respectively. Apart from the therapeutic approach based on newly identified and validated target molecules, the diagnostic approach is important as well. Such diagnostic approach is suitable to monitor the condition or disease to be treated, either prior to treatment or during or post treatment to allow the decision makers such as the medical doctors to decide on whether to proceed with the treatment or to adapt it to the needs of the individual patient.

The problem underlying the present invention was therefore to provide a target which is specific for cancers and tumors, respectively. A further problem underlying the present invention is to provide a target which is more particularly involved in tumorigenesis and metastasis. Finally it is a problem underlying the present invention to provide for a diagnostic marker related to tumorigenesis and metastasis.

These and other problems are solved by the subject matter of the independent claims. Preferred embodiments may be taken from the dependent claims.

International patent application WO 02/46465 discloses a polypeptide having the amino acid sequence according to SEQ.ID.No.2.

Shoshani et al., Molecular and Cellular Biology, vol.22, no. 7, pages 2283 - 2293 disclose the identification of a hypoxia-inducible factor-1 responsive gene.

In any aspect of the present invention related to a nucleic acid the nucleic acid may be present as DNA or RNA. It is also within the present invention that the nucleic acid may be present as single-stranded, partially double-stranded or double-stranded nucleic acid. In a preferred embodiment the nucleic acid according to the present invention is present as a single-stranded RNA. Such single-stranded RNA is preferably used as an mRNA in an expression system, whereby the expression system is preferably an expression system as described herein. In an embodiment of the nucleic acid according to the present invention the nucleic acid is single-stranded and would hybridise, but for the degeneracy of the genetic code, to a nucleic acid which is essentially complementary to the nucleic acid coding for the factor according to the present invention. In a further embodiment, the single-stranded nucleic acid according to the present invention hybridises under stringent conditions to a nucleic acid which is essentially complementary to the nucleic acid according to the second aspect of the present invention.

The present inventors have surprisingly found the polypeptide factor which is referred to herein as PI 3-kinase pathway regulated factor 1 or PRF1 involved in a number of biological processes namely, wound healing, metastasis, tumorigenesis, cell migration, cell motility in extracellular matrix and cell growth in extracellular matrix. These processes are under the control of the PI 3-kinase pathway.

The genomic sequence of PRF1 is localised on chromosome 10, more particularly the locus is 10 pter-q 26.12. The gene comprises a total of 1,760 nucleotides as currently defined. The respective databank entry which represents the cDNA, is referred to as NM_019058, more preferably NM_019058.1, and gi 9506686. The cDNA coding for PRF1 is referred to herein as SEQ ID NO. 2 and the coding sequence thereof as SEQ ID NO. 3. It is within the present invention that the nucleic acid coding for PRF1 may have some variations such as at position 312, where C is replaced by G, at position 991, where A is replaced by T, at position 1247, where C is replaced by T, at complement position 1297, where C is replaced by T, at position 1958, where C is replaced by T, at complement position 1666, where A is replaced by G, and at position 1743, where A is replaced by C. All of the aforementioned positions refer to the cDNA which is also referred to herein as SEQ ID NO.2. The open reading frame starts at position 198 and ends at position 896.

The amino acid sequence of PRF1 is also referred to herein as SEQ ID NO. 1. The respective databank entry which represents the amino acid sequence is NP_061931, more particularly NP_061931.1, and gi 9506687.

The polypeptide according to the present invention may also comprise variations such as, e. g., at amino acid position 39 where R is replaced by G.

The polypeptide according to the present invention has recently been referred to in the literature as REDD1, and is as such described by Ellisen, L. W., Molecular Cell, Vol. 10, 995-1005, November, 2002.

It is to be understood that there may be some sequencing errors contained in any of said sequences, however, it will be acknowledged by the ones skilled in the art that the basic sequence is the one specified above which may be further matured and from the above accession numbers a respective amended sequence may be taken which shall thus also be comprised by the scope of this invention. Also comprised by the scope of the present invention shall be embodiments where one or more of the above variations are contained or realised.

It is within the present invention that derivatives or truncated versions of PRF1 or nucleic acids coding for the same may be used according to the present invention as long as the desired effects may be realised. The desired effects may, among others, be the one that the particular compounds such as functional nucleic acids, antibodies, polypeptides and small molecules may still be screened or designed. Insofar the term PRF1 also comprises this kind of derivatives or truncated versions. A preferred derivative of PRF1 is, among others, a phosphorylated version or a glycosylated version thereof. The extent of derivatisation and truncation can thus be determined by the ones skilled in the art by routine analysis. When it comes to nucleic acid sequences those nucleic acid sequences are also comprised by the term nucleic acid sequences encoding PRF1 which are hybridising to the nucleic acid specified by the aforementioned accession numbers or any nucleic acid sequence which may be derived from the aforementioned amino acid sequences. Such hybridisation and the experimental particularities thereof are known to the one skilled in the art. The particularities of such hybridisation may, e. g., also be taken from Ausubel et al., (1996) Current Protocols in Molecular Biology. J. Wiley and Sons, New York. Stringent hybridization as used herein means, e.g., 0,1xSSC; 0,1%SDS, 65oC, 15 min. In addition, the term nucleic acid coding for PRF1 comprises also a nucleic acid sequence which is homologous to any of the aforementioned nucleic acid sequences, whereby the degree of homology is preferably 75, 80, 85, 90 or 95 % including any percentage between 70 % and 99 %.

The gene coding for PRF1 is described in literature without annotating any function to it. It is stated that the gene is hypoxia-inducible and responsive to Hypoxia inducible factor 1 (Shoshani T., et al., Molecular and cellular biology, April 2002, p. 2283 - 2293).

In said paper PRF1 is said to be regulated by hypoxia. However, it is not disclosed there that PRF1 is a survival factor which is related to and involved in the hypoxia response. The present inventors have surprisingly found that PRF1 is regulated in a PI 3-kinase and/or HIF1α dependent manner. Knockdown of PRF1 is suitable to inhibit the growth of the cells in a matrigel assay as described in the examples herein. In connection therewith, it is noteworthy that the knockdown of PRF1 can also be shown at the protein level and loss of function shows the inhibitory phenotype similarly to the effect of LY294002 confirming the inventors' finding that PRF1 is a specific target, more particularly a cancer target, which is downstream of HIF1α and Akt. This is the more relevant as there are numerous data available which confirm that HIF is highly relevant in tumor therapy. Also, knockdown of PRF1 results in an inhibition of a tumor as illustrated using PC-3 cells as a tumor model. From this various applications of PRF1 and more particularly of compounds inhibiting PRF1 may be derived. Accordingly, PRF1 may as such be overexpressed in a cellular system in order to allow the cells to survive hypoxic conditions. Such conditions are, e. g., realised in a stroke, heart failure, so that overexpression or activation of PRF1 might be a suitable means for the treatment or prevention of this kind of diseases. It will be acknowledged by the ones skilled in the art that any compound or mechanism involved in the increased expression or activity of PRF1 may thus be a suitable means for the treatment and/or prevention of this kind of disease as well.

Furthermore, it is within the present invention that any decrease in the expression or, as used herein in a synonymous manner, activity of PRF1 is suitable to put a respective cellular system into a condition corresponding to hypoxic conditions. Under these conditions further cellular process such as, e. g., apoptosis may start. By inhibiting PRF1 hypoxic conditions may be realised which in turn are suitable to treat those diseases where it is intended that the cells shall not be provided with enough oxygen. There are diseases known which can be treated by conferring the cells involved into a condition which said cells experience or are exposed to or would experience under hypoxia. One such disease are the tumors and cancers as described herein. Insofar, any compound, particularly those described herein, which is/are suitable to decrease the expression and/or activity of PRF1 both at the transcription level and the translation level may be suitable for the prevention and/or treatment of this kind of disease. These diseases are also referred to herein as hypoxia related diseases. These compounds or means may be effective in the treatment of, among others, tumors based on inhibition of tumor angiogenesis.

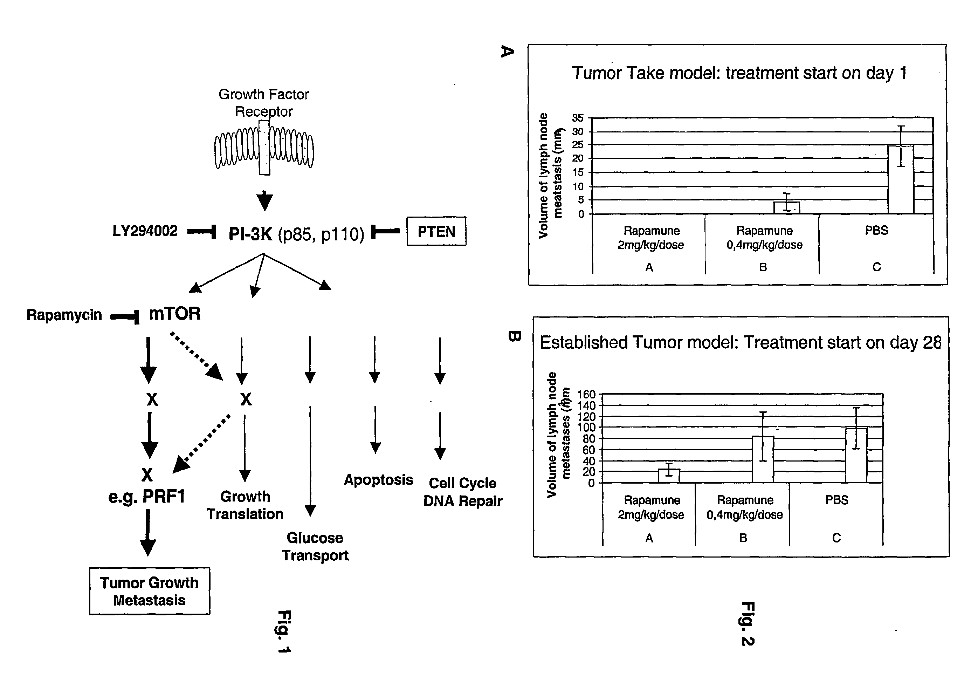

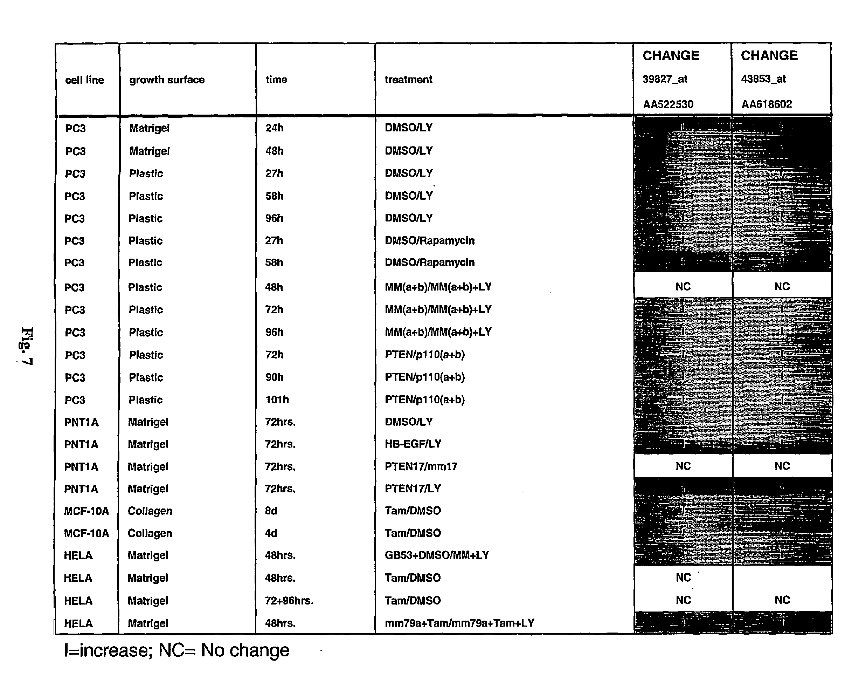

The present inventors have surprisingly found that PRF1 is a valuable target in connection with cancer and tumours and other biological processes such as metastasis, tumorigenesis, cell migration, cell motility in extracellular matrix and cell growth in extracellular matrix and any diseases and diseased conditions based thereon or involving any of these processes. More particularly, the present inventors have discovered that PRF1 is a downstream target of the PI-3 kinase/PTEN pathway and/or the HIF1α pathway. Even more surprisingly the present inventors have discovered that PRF1 is linked to several of the branches of the PI 3-kinase pathway as depicted in Fig. 1 herein. Additionally, the present inventors have surprisingly discovered that PRF1 is highly regulated through the HIF1α pathway which parallels the Akt pathway as also depicted in Fig. 7. In connection with PRF1 the branch related to metastasis and migration are most relevant. The importance of PRF1 in these processes is shown in case of the loss of suppressor function, more particularly PTEN tumour suppressor function. As will be shown in the examples, PRF1 will be up-regulated under conditions where PTEN which is an inhibitor to the PI-3 kinase pathway, is not active.

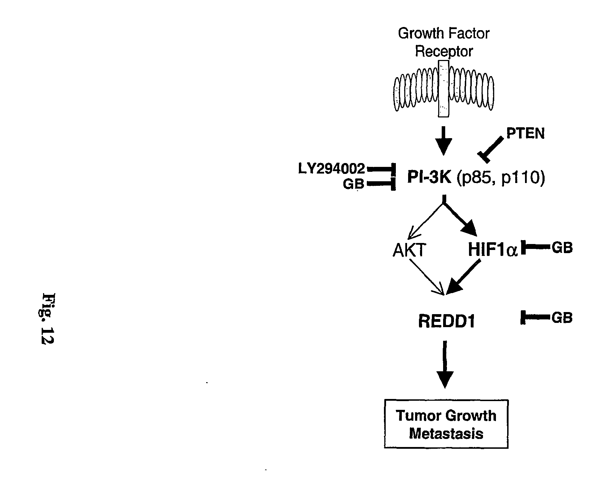

PRF1 is a valuable diagnostic marker the expression of which is increased in PTEN minus cells which are characteristic for a number of late stage tumors and diseased conditions (Cantley, L. C. and Neel, B. G. (1999). Proc. Natl. Acad. Sci. USA 96, 4240 - 4245; Ali, I. U. (2000). I. Natl. Cancer Inst. 92, 861 - 863). Also, loss of PTEN correlates with increased aggressive and invasive behaviour of the respective tumor cells. Accordingly, PRF1 is a valuable diagnostic agent in relation thereto. On the other hand, these results also indicate that PRF1 is a valuable downstream drug target of the PI 3-kinase/PTEN pathway and/or the HIF1α pathway which may be addressed accordingly by the different therapeutical approaches and respective agents as will be also disclosed herein. This means that an inhibitor of PRF1 is a suitable means for controlling metastatic and migrational behaviour of cells and this is a suitable means for the treatment of tumors and cancers, more particularly those tumors and cancers which are metastatic and the cells of which show a metastatic and/or migrational behaviour which are generally referred to herein as 'the disease as described herein' or as 'diseased condition as described herein'. The disease as described herein as well as the diseased condition as described herein also comprise tumorigenesis and metastasis, but are not limited thereto. Further diseases and diseased conditions, respectively, are generally those related to any disease or pathological condition involving the PI 3-kinase pathway and/or the HIF1α pathway and more particularly those different processes depicted in Fig. 1 and Fig. 12. One of these diseases related to the PI 3-kinase pathway and/or the HIF1α pathway is, among others, diabetes. This applies particularly to those diseases as described herein and those diseased conditions as described herein where the cells involved in such diseases or diseased conditions are PTEN negative which means that the tumor suppressor PTEN is not active or has a reduced level of activity or those in which the PI 3-kinase pathway and/or the HIF1α pathway is involved. Besides metastatic tumors diabetes belongs to this kind of diseases and diseased conditions, respectively. Therefore, cells, particularly those which are involved in the disease or diseased condition as described herein and which are PTEN negative, are susceptible to the treatment by a drug the mode of action is such as to reduce or eliminate the activity of PRF1 in the respective cells involved. Accordingly, patients whose tumors are PTEN negative or who have cells which are PTEN negative, particularly if these cells are involved in the disease as described herein or in the diseased condition as described herein, can advantageously be treated using said drugs. The same applies also to respective diagnostic agents or medicaments.

A further group of patients who can advantageously be treated using said drugs are those who suffer from cancers which have a high incidence for loss of PTEN function, especially in late stage tumors (Cantley, L. C. and Neel, B. G. (1999). New insights into tumor suppression: PTEN suppresses tumor formation by restraining the phosphoinositide 3-kinase/AKT pathway and/or the HIF1α pathway. Proc Natl Acad Sci U S A 96, 4240-4245; Ali, I. U. (2000). Gatekeeper for endometrium: the PTEN tumor suppressor gene. J Natl Cancer Inst 92, 861-863). Loss of PTEN correlates with increased aggressive and invasive behavior of the respective tumor cells. Because of this, in preferred embodiments those diagnostic agents and therapeutic agents, respectively, directed to PRF1 can be used for any tumor provided that the aforementioned prerequisite is met, namely that PTEN correlates with increased aggressive and invasive behaviour.

In the light of the disclosure given herein, namely that PRF1 is a downstream target of the PI 3-kinase pathway and/or the HIF1α pathway, but also to proliferation control, metastasis and migration, the one skilled in the art may develop both therapeutical agents and diagnostic agents for the diseases described herein and diseased conditions described herein, respectively. A representative description on how drugs may be screened once a target validated is, e. g., described by Prendergast, Nature Biotechnology, October 2001, Vol. 19, p. 919 - 921 or Torrance C. J. et al., Nature Biotechnology, October 2001, Vol. 19, p. 940 - 945.

Because of the involvement of PRF1 in the mechanisms as outlined above, it or a nucleic acid coding for it can also be used as a marker for diagnosing the status of a cell or patient having in his body such kind of cell, whether it will undergo metastasis, tumorigenesis or any other process described herein and whether it is suffering from oxygen, glucose and/or glucose depletion which in turn is indicative for tumor cells, particularly for fast growing cancer cells. As an example that this kind of approach works and is applicable for that purpose is, e. g., ICAM-1. ICAM-1 is used in the prognosis of gastric cancers to undergo metastasis (Maruo Y, Gochi A, Kaihara A, Shimamura H, YamadaT, TanakaN, OritaK.Int J Cancer. 2002 Aug 1;100(4):486-490 ) where s-ICAM-1 levels were found to be elevated in patients with liver metastasis. In another example, osteopontin is used as a prognostic marker for breast cancer (Rudland PS, Platt-Higgins A, E1-Tanani M, De Silva Rudland S, Barraclough R, Winstanley JH, Howitt R, West CR.Cancer Res. 2002 Jun 15;62(12):3417-3427.) In so far the presence or the level of presence (protein or mRNA) or the level of activity of PRF1 may be used as a marker and any compound more or less specifically interacting with PRF1 will therefore be an appropriate diagnostic agent and/or an appropriate analytical tool or means.

Methods and design principles for drugs and diagnostic agents which in any case specifically and/or selectively interact with PRF1 will be disclosed in the following.

In the light of these findings PRF1 proves to be a suitable downstream drug target which allows the selective modulation of only some aspects which are typically related to PI-3 kinase pathway, such as metastasis and migration as well as hypoxia response, cell growth, and wound healing, and a selective and specific diagnostic approach, i. e. detection, of processes typically related to PI 3-kinase pathway, more particularly metastasis and migration.

The PI 3-kinase pathway is characterized by a PI 3-kinase activity upon growth factor induction and a parallel signalling pathway. Growth factor stimulation of cells leads to activation of their cognate receptors at the cell membrane which in turn associate with and activate intracellular signalling molecules such as PI 3-kinase. Activation of PI 3-kinase (consisting of a regulatory p85 and a catalytic p110 subunit) results in activation of Akt by phosphorylation and/or the activation of HIF1α, thereby supporting cellular responses such as proliferation, survival or migration further downstream. PTEN is thus a tumor suppressor which is involved in the phosphatidylinositol (PI) 3-kinase pathway and which has been extensively studied in the past for its role in regulating cell growth and transformation (for reviews see, Stein, R. C. and Waterfield, M. D. (2000). PI3-kinase inhibition: a target for drug development? Mol Med Today 6, 347-357; Vazquez, F. and Sellers, W. R. (2000) and/or the HIF1α pathway. The PTEN tumor suppressor protein: an antagonist of phosphoinositide 3- kinase signaling. Biochim Biophys Acta 1470, M21-35; Roymans, D. and Slegers, H. (2001). Phosphatidylinositol 3-kinases in tumor progression. Eur J Biochem 268, 487-498). The tumor suppressor PTEN functions as a negative regulator of PI 3-kinase and/or the HIF1α pathway by reversing the PI 3-kinase-catalyzed reaction and thereby ensures that activation of the pathway occurs in a transient and controlled manner. Chronic hyperactivation of PI 3-kinase and/or the HIF1α pathway signalling is caused by functional inactivation of PTEN. PI 3-kinase activity and/or the HIF1α activity can be blocked by addition of the small molecule inhibitor LY294002. The activity and downstream responses of the signalling kinase MEK which acts in a parallel pathway, can, for example, be inhibited by the.small molecule inhibitor PD98059.

An activation particularly a chronic activation of the PI 3-kinase pathway and/or HIF1α activity through loss of PTEN function is a major contributor to tumorigenesis and metastasis indicating that this tumor suppressor represents an important checkpoint for a controlled cell proliferation.

PTEN knock out cells show similar characteristics as cells in which the PI 3-kinase pathway and/or the HIF1α pathway has been induced, particularly chronically induced, via activated forms of PI 3-kinase (Di Cristofano, A., Pesce, B., Cordon-Cardo, C. and Pandolfi, P. P. (1998). PTEN is essential for embryonic development and tumour suppression. Nat Genet 19, 348-355. Klippel, A., Escobedo, M. A., Wachowicz, M. S., Apell, G., Brown, T. W., Giedlin, M. A., Kavanaugh, W. M. and Williams, L. T. (1998). Activation of phosphatidylinositol 3-kinase is sufficient for cell cycle entry and promotes cellular changes characteristic of oncogenic transformation. Mol Cell Biol 18, 5699-5711. Kobayashi, M., Nagata, S., Iwasaki, T., Yanagihara, K., Saitoh, I., Karouji, Y., Ihara, S. and Fukui, Y. (1999). Dedifferentiation of adenocarcinomas by activation of phosphatidylinositol 3-kinase. Proc Natl Acad Sci U S A 96, 4874-4879).

The various diseases as described herein may also be characterised by a hyperactivation of the PI 3-kinase pathway and/or activation, preferably hyperactivation of the HIF1α pathway. This activation or hyperactivation is similar to the situation of a cell which lacks PTEN activity. Hyperactivation of the PI 3-kinase pathway and/or activation of the HIF1α pathway as used herein, particularly means an increased activity of the PI 3-kinase pathways and/or the HIF1α pathways compared to the activity of said pathways normally observed, i. e. the activity of the PI 3-kinase pathway of the particular kind of cell whereby the cell is not part of or involved in the disease or diseased condition.

PTEN is involved in several pathways which are also referred to as PTEN related pathways such as the PI3K/PTEN pathway, the Akt pathway, the EGF-related autocrine loop, the mTOR pathway and, as documented by the present inventors, also the HIF1α pathway. A PI3 - kinase pathway is actually any pathway which involved PI 3-kinase, either directly or indirectly. PI 3-kinase may act either as an inhibitor or as an activator in such pathway, or it may as such be regulated by other elements of the pathway.

There is ample of prior art describing diseases and conditions involving the PI 3-kinase pathway and/or the HIF1α pathway. Any of these conditions and diseases may thus be addressed by the inventive methods and the drugs and diagnostic agents the design, screening or manufacture thereof is taught herein. For reasons of illustration but not limitation it is referred to the following: colorectal carcinomas, gliomas, endometrial cancers, adenocarcinomas, endometrial hyperplasias, Cowden's syndrome, breast-ovarian cancer, prostate cancer (Ali, I. U., Journal of the National Cancer Institute, Vol. 92, no. 11, June 07, 2000, page 861 - 863), Bannayan-Zonana syndrome, LDD (Lhermitte-Duclos' syndrome) (Macleod, K., supra) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies (Vazquez, F., Sellers, W. R., supra).

In view of this, PRF1 is a valuable downstream drug target of the PI 3-kinase pathway and/or of the HIF1α pathway which can be addressed by drugs which will have less side effects than other drugs directed to targets upstream of PRF1. Insofar the present invention provides a drug target which is suitable for the design, screening, development and manufacture of pharmaceutically active compounds which are more selective than those known in the art, such as, for example,LY 294002. By having control over this particular fraction of effector molecules, i.e. PRF1 and any further downstream molecule involved in the pathway, only a very limited number of parallel branches thereof or further upstream targets in the signalling cascade are likely to cause unwanted effects. Therefore, the other activities of the PI-3 kinase/PTEN pathway and/or of the HIF1α pathway related to cell cycle, DNA repair, apoptosis, glucose transport, translation will not be influenced.

Apart from being a valuable target molecule in connection with the above specified diseases and regulatory networks, PRF1 as described herein, both at the nucleic acid level and at the protein level, is also a particularly valuable target in connection with the development or application of a combination therapy and medicaments used for such combination therapy. In other words, PRF 1, i.e. the nucleic acid coding therefore as well as the protein, can be used for the screening, generation, manufacture or design of a compound which can be used in sensitising a cell, tissue, organ or a patient such that the cell, tissue, organ or patient is susceptible to a treatment, preferably a treatment using a compound or medicament which is different from a/the compound and medicament, respectively, addressing PRF1 either at the nucleic acid level or at the protein level. It is within the scope of the present invention that said compound is also used for the manufacture of a medicament for the treatment of any of the conditions and diseases, respectively, as described herein, either alone or together with the compound and medicament, respectively, addressing PRF1.

Without wishing to be bound by any theory, it seems that PRF1 is regulated through both the HIF1alpha branch as well as the AKT branch of the PI 3-kinase pathway. As HIF1alpha is up-regulated under hypoxic conditions and Akt up-regulated under stress conditions to trigger a survival response which is counter-acting the apoptosis reaction of a cell, a compound addressing PRF1 as described herein and which may also be used as medicament or interaction partner of PRF1, both at the nucleic acid level and/or the protein level, can transfer the cell into a condition, whereupon other compounds are particularly effective. It is therefore within the scope of the present invention to use said PRF1 addressing compounds for both the screening of compounds which can be used for the treatment of any of the diseases described herein, including but not limited to the tumor diseases, and for the treatment of said diseases. Therefore, the screening can, for example, be carried out under hypoxic conditions and/or applying stress to a cell, such as applying cytostatics, e.g., cis platinum, radiation, preferably as used in connection with the treatment of tumors, and hyperthermia. Preferably, there are thus two targets involved in the screening, generation, manufacture and/or design process for a compound and medicament, respectively, for the treatment and/or development of a medicament for the treatment of any of the diseases disclosed herein, whereby one of said targets is preferably different from PRF1.

That the screening against a transcription factor such as HIF1α can successfully be performed, can be taken from Welsh, S. J. et al., Molecular Cancer Therapeutics Vol. 2, 235-243, March 2003.

Also, the insulin signalling is not induced which means that the diabetic responses or other side effects observed in connection with the use of LY294002 are actually avoided. LY294002 (2-(4-morpholinyl)8-phenylchromone) is one of several chromone derivatives small molecule inhibitor developed by Lilly Research Laboratories (Indianapolis) as an inhibitor for PI-3K (Vlahos et al. 1994, JBC 269, 5241 - 5248). It targets their catalytic subunit of the PI-3K molecule, p110 and functions by competing with ADP binding in the catalytic centre. However, LY294002 cannot distinguish between different isoforms of p110 (alpha, beta, gamma, delta) which are suggested to have different cellular functions.

PRF1 is also further downstream of mTOR which is addressed by rapamycin. mTOR (mammalian Target Of Rapamycin), also known as Raft or FRAP, is acting downstream of PI 3-kinase to regulate processes such as the pp70 S6 kinase dependent entry into the cell cycle. mTOR acts as a sensor for growth factor and nutrient availability to control translation through activating pp70 S6 kinase and initiation factor 4E. mTOR function is inhibited by the bacterial macrolide rapamycin which blocks growth of T-cells and certain tumor cells (Kuruvilla and Schreiber 1999, Chemistry & Biology 6, R129-R136).

The fact that rapamycin and its derivatives are suitable drugs currently being used in the clinic proves that a drug target is the more helpful and has the less side effects, the more specific it is for a particular molecular mechanism as, e. g. demonstrated by Yu et al. (Yu, K. et al (2001) Endrocrine-RelatCanc 8, 249). Since rapamycin is used for immunosuppression in human beings drugs interfering with PRF1 might be even more specific for this indication.

Because of the specificity of PRF1 outlined above in connection with its use as a potential drug target, it may also be used as diagnostic marker and respectively designed agents allowing preferably a selective and specific detection of PRF1 shall be used as diagnostic agents. Again, the closer and more specific a marker is to a certain biological phenomenon or in terms of signalling pathways, the closer it is to the finally observed effect, the more reliable is any statement on whether the final process is likely to occur which, in the present case, is metastasis and tumorigenesis, respectively. Therefore, diagnostic agents based on PRF1 and detecting PRF1, respectively, are diagnostic agents which allow a more reliable assessment of the likelihood of tumorigenesis and metastasis, respectively, or any of the other diseases described herein and diseased conditions described herein. These predictions are particularly related to those diseases described herein and diseased conditions described herein. In the design, screening and/or manufacture of therapeutic agents based on PRF1 as disclosed herein, PRF1 may be used as the compound against which chemical compounds which may be used as drugs or drug candidates or as diagnostic agents, are directed. These chemical compounds belong to different classes of compounds such as antibodies, peptides, anticalines, aptamers, spiegelmers, ribozymes, antisense oligonucleotides and siRNA as well as small molecules. The compounds are designed, selected, screened generated and/or manufactured by either using PRF1 itself as.a physical or chemical entity or information related to PRF1. In the design, selection, screening, generation and/or manufacturing process of said classes of compounds PRF1 will also be referred to as the target which is used in the process rather than in the final application of the respective compound to a patient in need thereof. In the processes which provide the various classes of compounds, either PRF1 or a nucleic acid coding PRF1 may be used including any embodiment thereof having or comprising any of the above described variations. The term PRF1 as used herein comprises any fragment or derivative of PRF1 which allows the design, selection, screening, generation and/or manufacture of said classes of compounds of the respective class(es) of compounds which in turn are/is upon their/its application as a medicament or as a diagnostic agent active as such. The term nucleic acid coding for PRF1 as used herein shall comprise any nucleic acid which contains a nucleic acid which codes PRF1 as defined above, or a part thereof. A part of a nucleic acid coding for PRF1 is regarded as such as long as it is still suitable for the design, selection, screening, generation and/or manufacture of said classes of compounds which in turn are/is upon their/its application as a medicament or as a diagnostic agent active as such. The nucleic acid coding for PRF1 may be genomic nucleic acid, hnRNA, mRNA, cDNA or a part of each thereof.

It is within the present invention that the chemical compounds as described above, i. e., but not limited thereto, antibodies, peptides, anticalines, aptamers, spiegelmers, ribozymes, antisense oligonucleotides and siRNA may be used for the same purposes as described for PRF1 as such.

As outlined above it is within the present invention that apart from PRF1 or a part or derivative thereof or a nucleic acid sequence therefore, as described herein, also other means or compounds may be used in order to create or to suppress the effects arising from PRF1 or the nucleic acid coding PRF1. Such means may be determined or selected in a screening method. In such screening method a first step is to provide one or several so called candidate compounds. Candidate compounds as used herein are compounds the suitability of which is to be tested in a test system for treating or alleviating the various diseases as described herein and diseased conditions as described herein or to be used as a diagnostic means or agent for this kind of diseases and diseased conditions. If a candidate compound shows a respective effect in a test system said candidate compound is a suitable means or suitable agent for the treatment of said diseases and diseased conditions and, in principle, as well as a suitable diagnostic agent for said diseases and diseased conditions. In a second step the candidate compound is contacted with a PRF1 expression system or a PRF1 activity system. The PRF1 activity system is also referred to herein as a system detecting the activity of PRF1.

A PRF1 expression system is basically an expression system which shows or displays the expression of PRF1, whereby the extent or level of expression basically may be changed. A PRF1 activity system is essentially an expression system whereby the activity or condition of activity is measured rather than the expression of PRF1. More particularly, it is tested whether under the influence of a candidate compound the activity of PRF1 or of the nucleic acid coding PRF1 is different from the situation without the candidate compound. Regardless whether the particular system is either an expression system or an activity system, it is within the scope of the present invention that either an increase or a decrease of the activity and expression, respectively, may occur and be measured. Typically, the expression system and/or activity system is an in vitro reaction system, such as a cell extract or a fraction of the cell extract such as a nucleus extract. A PRF1 expression system or activity system as used herein may also be a cell, a tissue or an organ, preferably a cell or a cell of a tissue or organ involved in the diseases as described herein and diseased conditions as described herein.

It is also within the present invention that the method for the screening of an agent according to the present invention may be performed such that after step d) the candidate compound obtained or identified in the first round of the sequence 1 steps inherent to the method, is subject to step c), whereby the expression system or activity system is different from the respective expression system or activity system used during the first round with which the candidate compound was characterised. Accordingly, in the first round the expression system and activity system, respectively, may be a cell involved in the diseases as described herein, whereas in the second round the cell may be a cell which is not involved in said diseases. In an alternative embodiment the order of use of the two cell types is reversed.

Whether there is an increase or decrease in the activity system or expression system may be determined at each level of the expression, for example by measuring the increase or decrease of the amount of nucleic acid coding for PRF1, more particularly mRNA or the increase or decrease of PRF1 expressed under the influence of the candidate compound. The techniques required for the measurement, more particularly the quantitative measurement of this kind of changes, such as for the mRNA or the protein are known to the one skilled in the art. Also known to the one skilled in the art are methods to determine the amount of or content of PRF1, e. g. by the use of appropriate antibodies. Antibodies may be generated as known to the one skilled in the art and described, e. g. by Harlow, E., and Lane, D., "Antibodies: A Laboratory Manual," Cold Spring Harbor Laboratory, Cold Spring Harbor, NY,(1988).

In case of a PRF1 expression system an increase or decrease of the activity of PRF1 may be determined, preferably in a functional assay.

Contacting the candidate compound and the expression system and activity system, respectively, usually is performed by adding an aqueous solution of the candidate compound to a respective reaction system which is generally referred to herein as test system. Besides aqueous solutions also suspensions or solutions of the candidate compound in organic solvents may be used. The aqueous solution is preferably a buffer solution.

Preferably, in each run using the expression system and activity system, respectively, only a single candidate compound is used. However, it is also within the present invention that several of this kind of tests are performed in parallel in a high throughput system.

A further step in the method according to the present invention resides in determining whether under the influence of the candidate compound the expression or activity of the expression system and activity system, respectively, in relation to PRF1 or a nucleic acid coding therefore is changed. Typically this is done by comparing the system's reaction upon addition of the candidate compound relative to the one without addition of the candidate compound. Preferably, the candidate compound is a member of a library of compounds. Basically any library of compounds is suitable for the purpose of this invention regardless of the class of compounds. Suitable libraries of compounds are, among others, libraries composed of small molecules, of peptides, proteins, antibodies, anticalines and functional nucleic acids. The latter compounds may be generated as known to the one skilled in the art and outlined herein.

The manufacture of an antibody specific for PRF1 or for the nucleic acid coding for PRF1, is known to the one skilled in the art and, for example, described in Harlow, E., and Lane, D., "Antibodies: A Laboratory Manual," Cold Spring Harbor Laboratory, Cold Spring Harbor, NY,(1988). Preferably, monoclonal antibodies may be used in connection with the present invention which may be manufactured according to the protocol of Cesar and Milstein and further developments based thereon. Antibodies as used herein, include, but are not limited to, complete antibodies, antibody fragments or derivatives such as Fab fragments, Fc fragments and single-stranded antibodies, as long as they are suitable and capable of binding to protein kinase N beta. Apart from monoclonal antibodies also polyclonal antibodies may be used and/or generated. The generation of polyclonal antibodies is also known to the one skilled in the art and, for example, described in Harlow, E., and Lane, D., "Antibodies: A Laboratory Manual," Cold Spring Harbor Laboratory, Cold Spring Harbor, NY,(1988). Preferably, the antibodies used for therapeutical purposes are humanized or human antibodies as defined above.

The antibodies which may be used according to the present invention may have one or several markers or labels. Such markers or labels may be useful to detect the antibody either in its diagnostic application or its therapeutic application. Preferably the markers and labels are selected from the group comprising avidine, streptavidine, biotin, gold and fluorescein. These and further markers are described in Harlow et al. (Harlow, E., and Lane, D., "Antibodies: A Laboratory Manual," Cold Spring Harbor Laboratory, Cold Spring Harbor, NY,(1988)).

It is also within the present invention that the label or marker exhibits an additional function apart from detection, such as interaction with other molecules. Such interaction may be, e.g., specific interaction with other compounds. These other compounds may either be those inherent to the system where the antibody is used such as the human or animal body or the sample which is analysed by using the respective antibody. Appropriate markers may, for example, be biotin or fluoresceine with the specific interaction partners thereof such as avidine and streptavidine and the like being present on the respective compound or structure to interact with the thus marked or labelled antibody.

A further class of medicaments as well as diagnostic agents which may be generated using the protein of PRF1 or the nucleic acid coding for PRF1, are peptides which bind thereto. Such peptides may be generated by using methods according to the state of the art such as phage display. Basically, a library of peptide is generated, such as in form of phages, and this kind of libraries is contacted with the target molecule, in the present case, for example, PRF1. Those peptides binding to the target molecule are subsequently removed, preferably as a complex with the target molecule, from the respective reaction. It is known to the one skilled in the art that the binding characteristics, at least to a certain extend, depend on the particularly realized experimental set-up such as the salt concentration and the like. After separating those peptides binding to the target molecule with a higher affinity or a bigger force, from the non-binding members of the library, and optionally also after removal of the target molecule from the complex of target molecule and peptide, the respective peptide(s) may subsequently be
characterised. Prior to the characterisation optionally an amplification step is realized such as, e. g. by propagating the peptide coding phages. The characterisation preferably comprises the sequencing of the target binding peptides. Basically, the peptides are not limited in their lengths, however, preferably peptides having a lengths from about 8 to 20 amino acids are preferably obtained in the respective methods. The size of the libraries may be about 10² to 10¹⁸, preferably 10⁸ to 10¹⁵ different peptides, however, is not limited thereto.

A particular form of target binding polypeptides are the so-called "anticalines" which are, among others, described in German patent application DE 197 42 706.

According to the present invention the PRF1 as well as the nucleic acid coding for PRF1 may be used as the target for the manufacture or development of a medicament for the treatment of the diseases described herein and of the diseased conditions described herein, as well as for the manufacture and/or development of means for the diagnosis of said diseases and said conditions, in a screening process, whereby in the screening process small molecules or libraries of small molecules are used. This screening comprises the step of contacting the target molecule with a single small molecule or a variety of small molecules at the same time or subsequently, preferably those from the library as specified above, and identifying those small molecules or members of the library which bind to the target molecules which, if screened in connection with other small molecules may be separated from the non-binding or non-interacting small molecules. It will be acknowledged that the binding and non-binding may strongly be influenced by the particular experimental set-up. In modifying the stringency of the reaction parameters it is possible to vary the degree of binding and non-binding which allows a fine tuning of this screening process. Preferably, after the identification of one or several small molecules which specifically interact with the target molecule, this small molecule may be further characterised. This further characterisation may, for example, reside in the identification of the small molecule and determination of its molecule structure and further physical, chemical, biological and/or medical characteristics. Preferably, the natural compounds have a molecular weight of about 100 to 1000 Da. Also preferably, small molecules are those which comply with the Lepinsky rules of five known to the ones skilled in the art. Alternatively, small molecules may also be defined such that they are synthetic small molecules, preferably arising from combinatorial chemistry, in contrast to natural products which preferably are non-synthetic. However, it is to be noted that these definitions are only subsidiary to the general understanding of the respective terms in the art.

It is also within the present invention to use PRF1 and/or a nucleic acid coding for PRF1 as a target molecule for the manufacture or selection of aptamers and spiegelmers which may then be used directly or indirectly either as medicament or as diagnostic agents.

Aptamers are D-nucleic acids which are either single stranded or double stranded and which specifically interact with a target molecule. The manufacture or selection of aptamers is, e. g., described in European patent EP 0 533 838. Basically the following steps are realized. First, a mixture of nucleic acids, i. e. potential aptamers, is provided whereby each nucleic acid typically comprises a segment of several, preferably at least eight subsequent randomised nucleotides. This mixture is subsequently contacted with the target molecule whereby the nucleic acid(s) bind to the target molecule, such as based on an increased affinity towards the target or with a bigger force thereto, compared to the candidate mixture. The binding nucleic acid(s) are/is subsequently separated from the remainder of the mixture. Optionally, the thus obtained nucleic acid(s) is amplified using, e. g. polymerase chain reaction. These steps may be repeated several times giving at the end a mixture having an increased ratio of nucleic acids specifically binding to the target from which the final binding nucleic acid is then optionally selected. These specifically binding nucleic acid(s) are referred to aptamers. It is obvious that at any stage of the method for the generation or identification of the aptamers samples of the mixture of individual nucleic acids may be taken to determine the sequence thereof using standard techniques. It is within the present invention that the aptamers may be stabilized such as, e. g., by introducing defined chemical groups which are known to the one skilled in the art of generating aptamers. Such modification may for example reside in the introduction of an amino group at the 2'-position of the sugar moiety of the nucleotides. Aptamers are currently used as therapeutical agens. However, it is also within the present invention that the thus selected or generated aptamers may be used for target validation and/or as lead substance for the development of medicaments, preferably of medicaments based on small molecules. This is actually done by a competition assay whereby the specific interaction between the target molecule and the aptamer is inhibited by a candidate drug whereby upon replacement of the aptamer from the complex of target and aptamer it may be assumed that the respective drug candidate allows a specific inhibition of the interaction between target and aptamer, and if the interaction is specific, said candidate drug will, at least in principle, be suitable to block the target and thus decrease its biological availability or activity in a respective system comprising such target. The thus obtained small molecule may then be subject to further derivatisation and modification to optimise its physical, chemical, biological and/or medical characteristics such as toxicity, specificity, biodegradability and bioavailability.

The generation or manufacture of spiegelmers which may be used or generated according to the present invention using PRF1 or a nucleic acid coding for PRF1, is based on a similar principle. The manufacture of spiegelmers is described in international patent application WO 98/08856. Spiegelmers are L-nucleic acids, which means that they are composed of L-nucleotides rather than D-nucleotides as aptamers are. Spiegelmers are characterized by the fact that they have a very high stability in biological system and, comparable to aptamers, specifically interact with the target molecule against which they are directed. In the process of generating spiegelmers, a heterogonous population of D-nucleic acids is created and this population is contacted with the optical antipode of the target molecule, in the present case for example with the D-enantiomer of the naturally occurring L-enantiomer of PRF1. Subsequently, those D-nucleic acids are separated which do not interact with the optical antipode of the target molecule. But those D-nucleic acids interacting with the optical antipode of the target molecule are separated, optionally determined and/or sequenced and subsequently the corresponding L-nucleic acids are synthesized based on the nucleic acid sequence information obtained from the D-nucleic acids. These L-nucleic acids which are identical in terms of sequence with the aforementioned D-nucleic acids interacting with the optical antipode of the target molecule, will specifically interact with the naturally occurring target molecule rather than with the optical antipode thereof. Similar to the method for the generation of aptamers it is also possible to repeat the various steps several times and thus to enrich those nucleic acids specifically interacting with the optical antipode of the target molecule.

A further class of compounds which may be manufactured or generating based on PRF1 or a nucleic acid coding for PRF1, as the target molecule as disclosed herein, are ribozymes, antisense oligonucleotides and siRNA. Due to the function and mode of action of PRF1 these kinds of molecules, which are also referred to and used as nucleic acid based drugs, are preferably created and/or manufactured based on the disclosure of the present invention, more preferably based on the nucleic acid sequences disclosed and described herein.

It is a common feature of all of the aforementioned nucleic acids that they do not interact with the target molecule at the level of the translation product which is in the present case PRF1, but rather interact with the transcription product, i. e. the nucleic acid coding for PRF1 such as the genomic nucleic acid or any nucleic acid derived therefrom such as the corresponding hnRNA, cDNA and mRNA, respectively. Insofar, the target molecule of the aforementioned class of compounds is preferably the mRNA of PRF1.

Ribozymes are catalytically active nucleic acids which preferably consist of RNA which basically comprises two moieties. The first moiety shows a catalytic activity whereas the second moiety is responsible for the specific interaction with the target nucleic acid, in the present case the nucleic acid coding for protein kinase N beta. Upon interaction between the target nucleic acid and the second moiety of the ribozyme, typically by hybridisation and Watson-Crick base pairing of essentially complementary stretches of bases on the two hybridising strands, the catalytically active moiety may become active which means that it catalyses, either intramolecularly or intermolecularly, the target nucleic acid in case the catalytic activity of the ribozyme is a phosphodiesterase activity. Subsequently, there may be a further degradation of the target nucleic acid which in the end results in the degradation of the target nucleic acid as well as the protein derived from the said target nucleic acid which in the present case is PRF1 due to a lack of newly synthesized PRF1 and a turn-over of prior existing PRF1. Ribozymes, their use and design principles are known to the one skilled in the art, and, for example described in Doherty and Doudna (Ribozym structures and mechanism. Annu ref. Biophys. Biomolstruct. 2001 ; 30 :457-75) and Lewin and Hauswirth (Ribozyme Gene Therapy: Applications for molecular medicine. 2001 7: 221-8).

The use of antisense oligonucleotides for the manufacture of a medicament and as a diagnostic agent, respectively, is based on a similar mode of action. Basically, antisense oligonucleotides hybridise based on base complementarity, with a target RNA, preferably with a mRNA, thereby activate RNase H. RNase H is activated by both phosphodiester and phosphorothioate-coupled DNA. Phosphodiester-coupled DNA, however, is rapidly degraded by cellular nucleases with the exception of phosphorothioate-coupled DNA. These resistant, non-naturally occurring DNA derivatives do not inhibit RNase H upon hybridisation with RNA. In other words, antisense polynucleotides are only effective as DNA RNA hybride complexes. Examples for this kind of antisense oligonucleotides are described, among others, in US-patent US 5,849,902 and US 5,989,912. In other words, based on the nucleic acid sequence of the target molecule which in the present case is the nucleic acid coding for PRF1, either from the target protein from which a respective nucleic acid sequence may in principle be deduced, or by knowing the nucleic acid sequence as such, particularly the mRNA, suitable antisense oligonucleotides may be designed base on the principle of base complementarity.

Particularly preferred are antisense-oligonucleotides which have a short stretch of phosphorothioate DNA (3 to 9 bases). A minimum of 3 DNA bases is required for activation of bacterial RNase H and a minimum of 5 bases is required for mammalian RNase H activation. In these chimeric oligonucleotides there is a central region that forms a substrate for RNase H that is flanked by hybridising "arms" comprised of modified nucleotides that do not form substrates for RNase H. The hybridising arms of the chimeric oligonucleotides may be modified such as by 2'-O-methyl or 2'-fluoro. Alternative approaches used methylphosphonate or phosphoramidate linkages in said arms. Further embodiments of the antisense oligonucleotide useful in the practice of the present invention are P-methoxyoligonucleotides, partial P-methoxyoligodeoxyribonucleotides or P-methoxyoligonucleotides.

Of particular relevance and usefulness for the present invention are those antisense oligonucleotides as more particularly described in the above two mentioned US patents. These oligonucleotides contain no naturally occurring 5'→3'-linked nucleotides. Rather the oligonucleotides have two types of nucleotides: 2'-deoxyphosphorothioate, which activate RNase H, and 2'-modified nucleotides, which do not. The linkages between the 2'-modified nucleotides can be phosphodiesters, phosphorothioate or P-ethoxyphosphodiester. Activation of RNase H is accomplished by a contiguous RNase H-activating region, which contains between 3 and 5 2'-deoxyphosphorothioate nucleotides to activate bacterial RNase H and between 5 and 10 2'- deoxyphosphorothioate nucleotides to activate eucaryotic and, particularly, mammalian RNase H. Protection from degradation is accomplished by making the 5' and 3' terminal bases highly nuclease resistant and, optionally, by placing a 3' terminal blocking group.

More particularly, the antisense oligonucleotide comprises a 5' terminus and a 3' terminus; and from 11 to 59 5'→3'-linked nucleotides independently selected from the group consisting of 2'-modified phosphodiester nucleotides and 2'-modified P-alkyloxyphosphotriester nucleotides; and wherein the 5'-terminal nucleoside is attached to an RNase H-activating region of between three and ten contiguous phosphorothioate-linked deoxyribonucleotides, and wherein the 3'-terminus of said oligonucleotide is selected from the group consisting of an inverted deoxyribonucleotide, a contiguous stretch of one to three phosphorothioate 2'-modified ribonucleotides, a biotin group and a P-alkyloxyphosphotriester nucleotide.

Also an antisense oligonucleotide may be used wherein not the 5' terminal nucleoside is attached to an RNase H-activating region but the 3' terminal nucleoside as specified above. Also, the 5' terminus is selected from the particular group rather than the 3' terminus of said oligonucleotide.

Suitable and useful antisense oligonucleotides are also those comprising a 5' terminal RNase H activating region and having between 5 and 10 contiguous deoxyphosphorothioate nucleotides; between 11 to 59 contiguous 5'→3'-linked 2'-methoxyribonucleotides; and an exonuclease blocking group present at the 3' end of the oligonucleotide that is selected from the group consisting of a non-5'-3'-phosphodiester-linked nucleotide, from one to three contiguous 5'-3'-linked modified nucleotides and a non-nucleotide chemical blocking group.

Two classes of particularly preferred antisense oligonucleotides can be characterized as follows:

The first class of antisense oligonucleotides, also referred to herein as second generation of antisense oligonucleotides, comprises a total of 23 nucleotides comprising in 5' → 3' direction a stretch of seven 2'-O-methylribonucleotides, a stretch of nine 2'-deoxyribonucleotides, a stretch of six 2'-O-methylribonucleotides and a 3'-terminal 2'-deoxyribonucleotide. From the first group of seven 2'-O-methylribonucleotides the first four are phosphorothioate linked, whereas the subsequent four 2'-O-methylribonucleotides are phosphodiester linked. Also, there is a phosphodiester linkage between the last, i. e. the most 3'-terminal end of the 2'-O-methylribonucleotides and the first nucleotide of the stretch consisting of nine 2'-deoxyribonucleotides. All of the 2'-deoxyribonucleotides are phosphorothioate linked. A phosphorothioate linkage is also present between the last, i. e. the most 3'-terminal 2'-deoxynucleotide, and the first 2'-O-methylribonucleotide of the subsequent stretch consisting of six 2'-O-methylribonucleotides. From this group of six 2'-O-methylribonucleotides the first four of them, again in 5' → 3' direction, are phosphodiester linked, whereas the last three of them, corresponding to positions 20 to 22 are phosphorothioate linked. The last, i. e. terminal 3'-terminal 2'-deoxynucleotide is linked to the last, i. e. most 3'-terminal 2'-O-methylribonucleotide through a phosphorothioate linkage.

This first class may also be described by reference to the following schematic structure: RRRnnnuNNNNNNNNNnnnRRRN. Hereby, R indicates phosphorothioate linked 2'-O-methyl ribonucleotides (A, G, U, C); n stands for 2'-O-methyl ribonucleotides (A, G, U, C); N represents phosphorothioate linked deoxyribonucleotides (A, G, T, C).

The second class of particularly preferred antisense oligonucleotides, also referred to herein as third generation (of) antisense oligonucleotides or Gene Blocs, also comprises a total of 17 to 23 nucleotides with the following basic structure (in 5' → 3' direction).

At the 5'-terminal end there is an inverted abasic nucleotide which is a structure suitable to confer resistance against exonuclease activity and, e. g., described in WO 99/54459. This inverted abasic is linked to a stretch of five to seven 2'-O-methylribonucleotides which are phosphodiester linked. Following this stretch of five to seven 2'-O-methylribonucleotides there is a stretch of seven to nine 2'-deoxyribonucleotides all of which are phosphorothioate linked. The linkage between the last, i. e. the most 3'-terminal 2'-O-methylribonucleotide and the first 2'-deoxynucleotide of the 2'-deoxynucleotide comprising stretch occurs via a phosphodiester linkage. Adjacent to the stretch of seven to nine 2'-deoxynucleotides a stretch consistent of five to seven 2'-O-methylribonucleotides is connected. The last 2'-deoxynucleotide is linked to the first 2'-O-methylribonucleotide of the latter mentioned stretch consisting of five to seven 2'-O-methylribonucleotides occurs via a phosphorothioate linkage. The stretch of five to seven 2'-O-methylribonucleotides are phosphodiester linked. At the 3'-terminal end of the second stretch of five to seven 2'-O-methylribonucleotide another inverted abasic is attached.

This second class may also be described by reference to the following schematic structure: (GeneBlocs representing the 3rd generation of antisense oligonucleotides have also the following schematic structure:) cap-(nₚ)ₓ(Nₛ)_{y}(nₚ)_{z}-cap or cap-nnnnnnnNNNNNNNNNnnnnnnn-cap. Hereby, cap represents inverted deoxy abasics or similar modifications at both ends; n stands for 2'-O-methyl ribonucleotides (A, G, U, C); N represents phosphorothioate-linked deoxyribonucleotides (A, G, T, C); x represents an integer from 5 to 7; y represents an integer from 7 to 9; and z represents an integer from 5 to 7.

It is to be noted that the integers x, y and z may be chosen independently from each other although it is preferred that x and z are the same in a given antisense oligonucleotide. Accordingly, the following basic designs or structures of the antisense oligonucleotides of the third generation can be as follows: cap-(nₚ)₅(Nₛ)₇(nₚ)₅-cap, cap-(nₚ)₆(Nₛ)₇(nₚ)₅-cap, cap-(nₚ)₇(Nₛ)₇(nₚ)₅-cap, cap-(nₚ)₅(Nₛ)₈(nₚ)₅-cap, cap-(nₚ)₆(Nₛ)₈(nₚ)₅-cap, cap-(nₚ)₇(Nₛ)₈(nₚ)₅-cap, cap-(nₚ)₅(Nₛ)₉(nₚ)₅-cap, cap-(nₚ)₆(Nₛ)₉(nₚ)₅-cap, cap-(nₚ)₇(Nₛ)₉(nₚ)₅-cap, cap-(nₚ)₅(Nₛ)₇(Hₚ)₆-cap, cap-(nₚ)₆(Nₛ)₇(nₚ)₆-cap, cap-(nₚ)₇(Nₛ)₇(nₚ)₆-cap, cap-(nₚ)₅(Nₛ)₈(nₚ)₆-cap, cap-(nₚ)₆(Nₛ)₈(nₚ)₆-cap, cap-(nₚ)₇(Nₛ)₈(nₚ)₆-cap, cap-(nₚ)₅(Nₛ)₉(nₚ)₆-cap, cap-(nₚ)₆(Nₛ)₉(nₚ)₆-cap, cap-(nₚ)₇(Nₛ)₉(nₚ)₆-cap, cap-(nₚ)₅(Nₛ)₇(nₚ)₇-cap, cap-(nₚ)₆(Nₛ)₇(nₚ)₇-cap, cap-(nₚ)₇(Nₛ)₇(nₚ)₇cap, cap-(nₚ)₅(Nₛ)₈(nₚ)₇-cap, cap-(nₚ)₆(Nₛ)₈(nₚ)₇-cap, cap-(nₚ)₇(Nₛ)₈(nₚ)₇-cap, cap-(nₚ)₅(Nₛ)₉(nₚ)₇-cap, cap-(nₚ)₆(Nₛ)₉(nₚ)₇-cap and cap-(nₚ)₇(Nₛ)₉(nₚ)₇-cap.

A further class of compounds which may be generated based on the technical teaching given herein and which may be used as medicaments and/or diagnostic agents are small interfering RNA (siRNA) directed to the nucleic acid, preferably mRNA, coding for PRF1. siRNA is a double stranded RNA having typically a length of about 21 to about 23 nucleotides. The sequence of one of the two RNA strands corresponds to the sequence of the target nucleic acid such as the nucleic acid coding for PRF1, to be degraded. In other words, knowing the nucleic acid sequence of the target molecule, in the present case PRF1, preferably the mRNA sequence, a double stranded RNA may be designed with one of the two strands being complementary to said, e. g. mRNA of PRF1 and, upon application of said siRNA to a system containing the gene, genomic DNA, hnRNA or mRNA coding for PRF1, the respective target nucleic acid will be degraded and thus the level of the respective protein be reduced. The basic principles of designing, constructing and using said siRNA as medicament and diagnostic agent, respectively, is, among others, described in international patent applications WO 00/44895 and WO 01/75164.

Based on the mode of action of the aforementioned classes of compounds, such as antibodies, peptides, anticalines, aptamers, spiegelmers, ribozymes, antisense oligonucleotides as well as siRNA, it is thus also within the present invention to use any of these compounds targeting PRF1 and the nucleic acid coding therefore, respectively, for the manufacture of a medicament or a diagnostic agent for any of the diseases as described herein and any of the diseased conditions described herein. Furthermore, these agens may be used to monitor the progression of said diseases and diseased conditions and the success of any therapy applied, respectively.

The various classes of compounds designed according to the present invention such as antibodies, peptides, anticalines, small molecules, aptamers, spiegelmers, ribozymes, antisense oligonucleotides and siRNA may also be contained in a pharmaceutical composition. Preferably such pharmaceutical composition is used for the treatment of the diseases as described herein or the diseased conditions described herein. The pharmaceutical composition may comprise in an embodiment one or several of the aforementioned classes of compounds and/or one or more members of a single class, and optionally a further pharmaceutical active compound, and a pharmaceutically acceptable carrier. Such carrier may be either liquid or solid, for example a solution, a buffer, an alcoholic solution or the like. Suitable solid carriers are, among others, starch and the like. It is known to the one skilled in the art to provide respective formulations for the various compounds according to the aforementioned classes of compounds in order to realize the particular route of administrations such as oral, parenteral, subcutaneous, intravenous, intramuscular and the like.

The various compounds of the different classes of compounds as mentioned above, may also be, either alone or in combination, subject to or contained in a kit. Such kit comprises apart from the respective compound(s) additionally one or several further elements or compounds whereby the elements are selected from the group comprising buffers, negative controls, positive controls and instructions on the use of the various compounds. Preferably, the various compounds are present in either dry or liquid form, preferably as a unit dosage for a single administration each. The kit may particularly be used for the therapy, diagnosis or monitoring of the progress of the disease or applied therapies in relation to the diseases and diseased conditions as described herein.

In a further aspect PRF1 both the nucleic acid coding therefor and preferably the amino acid sequence and the PRF1 polypeptide or protein can be used for the development and/or generation and/or design of compounds which are useful in the treatment and/or prevention and/or diagnosis of any of the diseases and conditions described herein. For that purpose, in a first step an interaction partner of the PRF1, preferably of PRF1 protein is determined or screened. More preferably such interaction partner is a naturally occurring interaction partner and even more preferably such interaction partner is/are the natural interaction partners. The method for determining or screening such interaction partner are known to the ones skilled in the art. A well known technology is the so-called two-hybrid system which is described, e.g. in Ausubel (supra), unit 13.14 "Interaction Trap/Two-Hybrid System to Identify Interacting Proteins" or Fields S., Song, O., Nature. 1989 Jul 20; 340 (6230):245-6. An alternative thereof is to precipitate the PRF1 protein or a fragment thereof from cell extracts or cell lysates and determine which other factor is attached or co-precipitated with PRF1. Such analysis can be done using standard techniques known to the one skilled in the art such as mass spectroscopy. In a particular embodiment, the precipitation is an immune precipitation. In a further embodiment the precipitation is made using radio-labelled cells, preferably ³⁵S labelled cells. Apart from that interaction partners, particularly natural interaction partners can be determined by using chromatographic means and characterising the fraction obtained, i.e. eluted. In a preferred embodiment such chromatographic means can also be affinity chromatography, whereby PRF1 protein is immobilized on the separation medium. Due to specific interaction between the PRF1 protein and the possible interaction partner, this interaction partner will act as a ligand in the chromatography process and can thus be purified and further characterized.

In a second step the thus identified interaction partner can be used in a screening process as described herein for PRF1. Additionally or alternatively, the nucleic acid encoding the interaction partner, if it is a polylpeptide or a nucleic acid based interaction partner, can be used for the design and/or manufacture and/or generation of the classes of compounds defined herein such as, but not limited to, antibodies, peptides, anticalines, small molecules, aptamers, spiegelmers, ribozymes, antisense molecules (also referred to herein as antisense oligonucleotides), and siRNA. In connection with the term siRNA any form thereof shall be comprised, including but not limited to siNA as described in WO 03/070918.

It is to be understood that the above described first step can be a method of its own with the second step only being optionally performed.

The present invention is now further illustrated by the following figures, examples which are not intended to limit the scope of protection but are given for reasons of exemplification only. From said figures, examples further features, embodiments and advantages of the invention may be taken, wherein
- Fig. 1: shows a schematic representation of growth factor induced activation of the PI 3-kinase pathway;
- Fig. 2: shows a measurement of lymph node metastasis in an orthotopic PC-3 mouse model of the treatment with rapamycin (Rapamune);
- Fig. 3: shows the experimental approach to identify PRF1 as a downstream drug target of the PI 3-kinase pathway;
- Fig. 4: shows a primary GeneBloc screen on PC-3 cells;
- Fig. 5: shows the growth of PC-3 cells transfected with PRF1 specific GeneBlocs on matrigel with Fig. 5A showing the mRNA knock-down under these conditions and Fig. 5B showing photographs taken from the respective cells grown on matrigel;
- Fig. 6: shows the extent of prostate tumor growth inhibition by PRF1 specific RNA interference (Fig. 6C), and of total lymph node metastases upon using the same PRF1 specific RNA interference (Fig. 6D), with Fig. 6A showing the basic vector design for the expression of siRNA and Fig. 6B the siRNA sequences used;
- Fig. 7: shows the result of a differential expression experiment, whereby the expression of PRF1 was monitored in different cell lines grown on various growth surfaces with the cell being treated with different compounds so as to address different elements of the PI 3-kinase pathway;
- Fig. 8 A: shows the result of a Western blot analysis performed for characterising the specificity of a polyclonal antibody against PRF1;
- Fig. 8 B: shows two photographs of prostate cancer tissue stained with the polyclonal antibody characterized in Fig. 8A and using preimmune serum;
- Fig. 9 A: shows the result of a Western blot analysis of cells treated with LY294002 (LY) or DMSO at 24 h, 48 h, 72 h and 96 h;
- Fig. 9 B: shows the result of a Western blot analysis of PC-3 cells treated with an antisense molecule (GB) specific for p110β;
- Fig. 10 A: shows the result of a Western blot analysis of an experiment, whereby PC-3 cells were treated with an Akt specific antisense molecule (GB),
- Fig. 10 B: shows the result of a Western blot analysis of PC-3 cells grown under hypoxic conditions and treated with an antisense molecule specific for HIF1α;
- Fig. 11 A: shows photographs of PC-3 cells treated with various antisense molecules (GB) on extracellular matrix;
- Fig. 11 B: shows a Western blot analysis of the PC-3 cells shown in the photographs according to Fig. 5 A;
- Fig. 12: shows a schematic representation of growth factor induced activation of the PI 3-kinase pathway now comprising also HIF1α as a downstream element of the pathway parallel to Akt converging on RPF1, referred to as REDID1 therein; and

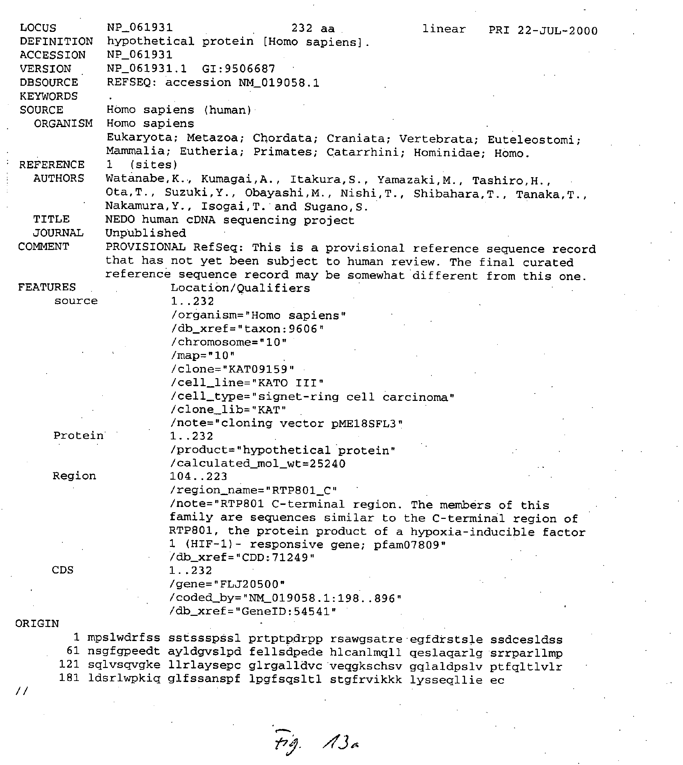

- Fig. 13a: depicts the amino acid sequence according to NP_061931.1; and

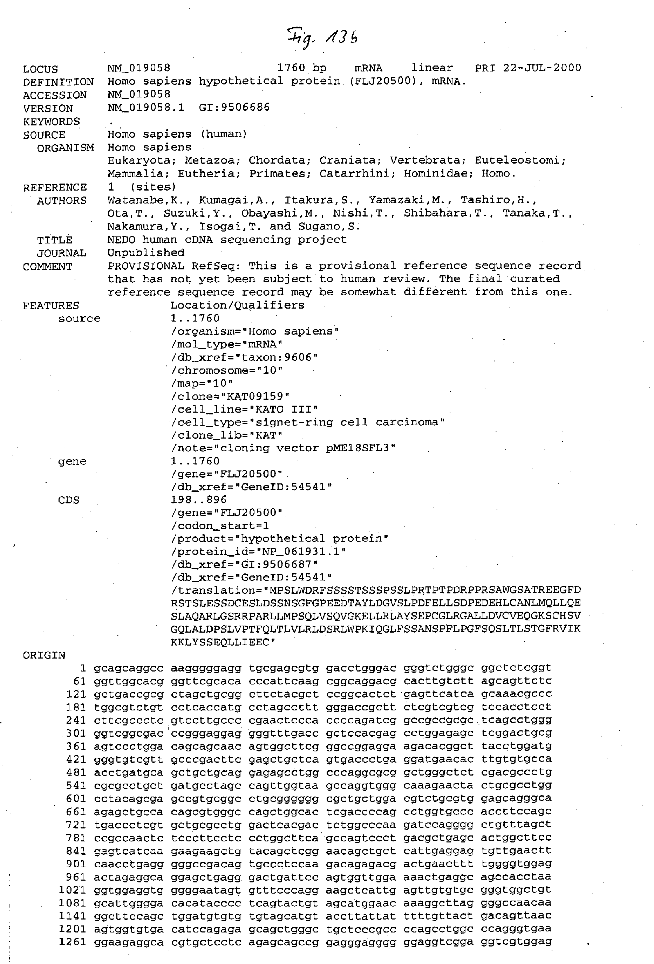

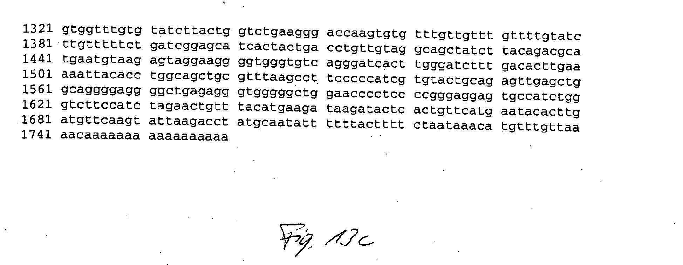

- Figs. 13b and c: depict the nucleic acid sequence according to NM_019058.1

Fig.1 shows a schematic representation of growth factor induced activation of the PI 3-kinase pathway. Growth factor stimulation of cells leads to activation of their cognate receptors at the cell membrane, which in turn associate with and activate intracellular signalling molecules such as PI 3-kinase. The tumor suppressor PTEN interferes with PI 3-kinase mediated downstream responses and ensures that activation of the pathway occurs in a transient manner. LY294002 is a small molecule inhibitor of PI 3-kinase. One of the known downstream genes of PI 3-kinase pathway is mTOR (mammalian target of Rapamycin) which can be inhibited by the clinically approved drug rapamycin (Rapamune). PI 3-K is involved in cell cycle and DNA repair, regulation of apoptosis, glucose transport, growth translation, metastasis and migration. X are indicating potential downstream drug targets that are supposed to be involved in promoting metastatic behavior of cancer cells and can be considered as better drug targets than more "upstream" targets such as mTOR due to reduced side effects.

Fig. 12 shows a schematic representation of growth factor induced activation of the PI 3-kinase pathway similar to the representation of Fig. 1. However, due to the findings underlying the present invention the PI 3-K pathway has been further elaborated such that HIF1α has been identified as a branch of the PI 3-K pathway to RPF1 which is parallel to the Akt mediated pathway.

A more detailed description of the other figures will be given in the following examples.

### Example 1: Materials and methods

### Cell culture

The human prostate carcinoma PC-3 cells were obtained from the American Type Culture Collection (ATCC). Cells were cultured in F12K Nutrient Mixture (Kaighn's modification) containing, 10 % fetal calf serum (CS), gentamycin (50 µg/ml) and amphotericin (50 ng/ml). Transfections were carried out in 96 well or 10-cm plates (at 30% to 50% confluency) by using various cationic lipids such as Oligofectamine, Lipofectamine (Life Technologies), NC388 (Ribozyme Pharmaceuticals, Inc., Boulder, CO), or FuGene 6 (Roche) according to the manufacturer's instructions. GeneBlocs were transfected by adding pre-formed 5x concentrated complex of GeneBloc and lipid in serum-free medium to cells in complete medium. The total transfection volume was 100 µl for cells plated in 96 wells and 10 ml for cells in 10 cm plates. The final lipid concentration was 0.8 to 1.2 µg/ml depending on cell density; the GeneBloc concentration is indicated in each experiment.

Cultivated cells were trypsinated and harvested following stopping the trypsin effect by medium. Washing procedures (PBS; Centrifugation 5min/1.000rpm) are added and, finally, the pellet is resuspended considering the cell number and volume to be inoculated.

### Determination of the relative amounts of RNA levels by Taqman analysis.

The RNA of cells transfected in 96-wells was isolated and purified using the Invisorb RNA HTS 96 kit (InVitek GmbH, Berlin). Inhibition of PRF1 mRNA expression was detected by real time RT-PCR (Taqman) analysis using 300 nM PRF1 5' primer, 300 nM PRF1 3' primer and 100 nM of the PRF1 Taqman probe Fam-Tamra labelled. The reaction was carried out in 50 µl and assayed on the ABI PRISM 7700 Sequence detector (Applied Biosystems) according to the manufacturer's instructions under the following conditions: 48°C for 30 min, 95°C for 10 min, followed by 40 cycles of 15 sec at 95°C and 1 min at 60°C.

### In vitro growth on matrigel matrix.

PC3 cells were tretated with 10µM LY294002 or DMSO when seeded on Matrigel. If cells were trnasfected previous to seeding cells were transfected with GeneBloc and trypsinized 48h post transfection. The cells were washed in medium and seeded into duplicate 24-wells (100.000 cells per well) pre-coated with 250 µl matrigel basement membrane matrix (Becton Dickinson). After incubation for 24 to 72 h photographs were taken at 5x magnification with an Axiocam camera attached to an Axiovert S100 microscope (Zeiss).

### Affymetrix

Total RNA from cells grown on Matrigel was prepared using Totally RNA kit (AMBION) following manufacturers protocol. In the final step precipitated total RNA was resuspended in Invisorb lysis buffer and purified using the Invisorb spin cell-RNA kit (INVITE). Biotin-labeled cRNA was prepared following Affymetrix protocols and 15µg cRNA were hybridized onto Affymetrix GeneChip set HG-U95.

### Data analysis

Raw data were analyzed using Affymetrix GeneChip software Microarray Suite v4.0. The intensity of each probe set is calculated as difference of the hybridization signal of perfect match oligonucleotides compared to mismatch oligonucleotides averaged over the set of 16 to 20 probe pairs corresponding to one transcript. The average difference of a probe set is proportional to the abundance of a transcript. Total signal intensities of different arrays were scaled to the same value before comparison. Fold changes were calculated using the Affymetrix software by pairwise comparison of the intensities of corresponding probe pairs from experiment and baseline arrays. Using decision matrices described by Affymetrix the software also generates absolute calls (transcript is absent, marginal or present in an experiment) and difference calls (abundance of a transcript in one experiment compared to another: increase, marginal increase, no change, marginal decrease, decrease). Results were exported to Microsoft Excel (absolute call, difference call, fold change) and filtered. All probe sets with absent calls or a no change call were discarded and the table sorted by the fold change.

### Animal studies

The in vivo experiments were conducted corresponding the Good Laboratory Practice for Nonclinical Laboratory Studies (GLP Regulations) of the Food and Drug Administration and in accordance with the German animal protection law as legal basis.

Male Shoe:NMRI-nu/nu mice (Tierzucht Schönwalde GmbH) maintained under SPF conditions (Laminar air flow equipment, Scantainer, Scanbur) served as recipients for the human prostate carcinoma cells. The animals, aged 6-8 weeks and weighing 28-30g, were inoculated 2x10⁶/0,03ml tumor cells into both, the left dorsolateral lobe of the prostatic gland (iprost; Orthotopic) or the tip of the Lobus lateralis sinister of the liver (ihep; Ectopic). For this purpose, the mice received a total body anaesthesia using a mixture of Ketanest (Parke-Davis GmbH) and Rompun (Bayer Vital GmbH) 80:1 with dosages of 100mg/kg and 5mg/kg, respectively. Following the thorough sterilization of the ventral body surface an incision was carried out through the abdominal skin and peritoneal wall beginning near the border of the preputial gland and measuring about 1cm. By means of a pair of tweezers and a cotton swab the prostatic gland was visualized. The orthotopic cell challenges followed with the help of a magnifying glas and by usage of a 1ml syringe (Henke Sass Wolf GmbH) bearing 30G 0,30x13 microlance needles (Becton Dickinson). An administration was successful observing a marked bleb at the inoculation site. The wound was closed by suture material (PGA Resorba, Franz Hiltner GmbH) concerning the peritoneal wall and Michel clamps 11x2mm (Heiland) for the abdominal skin. Wound spray (Hansaplast Sprühpflaster, Beiersdorf AG) covered the lesion. During the postsurgical phase the animals were maintained in a warmed environment until the complete waking up. The animals were randomised according to the number of treatment groups consisting of 5-10 animals per group each. They were inspected successively inclusive of protocolling the findings. Ssniff NM-Z, 10mm, autoclavable (ssniff Spezialdiäten GmbH) is administered as fortified diet and drinking water is acidified by HCl, both ad libitum.

### Evaluations

To receive the actual dosage level body weights were registered on the treatment days. At the same time, it can be derived from body weight development to recognize influences of treatment modalities on the whole organism.

Blood punctures were carried out on day 0 (Base line); 14; 28; and 35 (Sacrificing). Blood has been drawn from the orbital vein of the short term anaesthesized animal (Diethylether, Otto Fischar GmbH). Evaluation parameters giving data to the compatibility and side effects of the treatments are the following: Leukocyte numbers; Thrombocyte numbers; Enzymes. Further blood borne parameters (Bilirubin; Creatinine; Protein; Urea; Uric acid).

All sacrificed animals were completely dissected and photographically documented. Tumors (Prostatic gland) and metastases (Caudal, lumbar, renal lymph node metastases) were measured in two dimensions by means of a pair of callipers. The volume was calculated according to V (mm³) = ab²/2 with b < a . In general, the cell number performed for therapy approaches causes a 100% tumor take concerning the prostatic gland. The weights of some organs (Liver; Spleen; Kidney) were registered in order to find out additional data concerning the knowledge about secondary side effects.

For histological analysis samples of tumor tissues, i.e. prostate-tumor and lymph node metastases, were fixed in 5% formaldehyde and paraffin embedded. Routinely, the sections were HE stained, if necessary specific stainings were made (Azan, PAS).

To detect the human origin of tumor and metastatic cells adequate tissue samples were frozen in liquid nitrogen. When using PCR and Taqman analysis with huHPRT specific amplicon we could detect 50 human cells in 5mg tissue.

The therapeutic results were statistically verified by the u-test of Mann and Whitney.

### Example 2: Experimental proof-of-concept on the suitability of downstream drug targets

As outlined in the introductory part of this specification which is incorporated herein by reference, targets linked downstream to a signalling pathway are valuable for the design or development of both medicaments or drugs and diagnostic agents. It is obvious that, if the particular target is linked to different other pathways or, due to its position within the signalling pathway, to a number of biological phenomena such as, e. g. metastasis and migration, growth translation, apoptosis, cell cycle, DNA repair and the like as in the case of the PI-3 kinase pathway, any compound addressing this target is likely to have a number of side effects which may be detrimental to the system and undesired from the medical point of view, or cause a false or unspecific analytical result. Accordingly, downstream targets should be the first choice.

The present inventors have found that under the control of the PI 3-kinase pathway further possible targets apart from mTOR are involved, which are specific for controlling the phenomena of metastasis and migration and thus tumorigenesis and, possibly, also to growth translation and glucose and/or amino acid starvation. In the pharmaceutical industry it has been found that rapamycin, sold under the trade name of Rapamune is suitable to inhibit metastasis and migration as well as immunosuppression (e.g. for organ transplantation). This confirms the suitability of the strategy to address downstream drug targets.

As may be taken from Fig. 2 rapamycin is suitable to reduce the volume of lymph node metastasis and is insofar comparable in its effect to the well known PI 3-kinase inhibitor LY294002 which, however, is linked to number of side effects, which is not surprising given the fact that PI-3 kinase and mTOR are linked to a number of biological phenomena. As depicted in Fig. 2A the tumor take model was used and treatment with Rapamune started on day 1. Both concentrations used, i. e. 0.4 mg/kg/dose and 2mg/kg/dose led to a tremendous decrease of the extent of lymphnode metastasis, expressed as mm³ compared to the negative control which was phosphate buffered saline.

The same results were basically also obtained in case of Rapamune treatment of an established tumor model with the treatment starting on day 28 (Fig. 2B).

The underlying experimental set up was such that lymph node metastasis in an orthotopic PC-3 mouse model after treatment with rapamycin (Rapamune) was measured. In Fig. 2 (A) the results of the tumor take model are shown. Nude Shoe:NMRI -*nu*/*nu* mice (8 per group) were injected with 2x10⁶ PC3 cells in 0.03ml intraprostatic and treatment was carried out using Rapamune intraperitoneally daily for 28 days at doses of 2mg/kg and 0.4mg/kg. PBS served as a control.

For the treatment of established tumors (B), cells were allowed to grow ipros for 28 days and treatment was carried out orally using Rapamune on days 29 to 50 after implantation. Doses were chosen as outline in A. Animals were sacrificed on day 29 and 51, respectively and total lymph node metastasis were determined

### Example 3: Identification of PRF1 as downstream drug target of the PI 3-kinase pathway

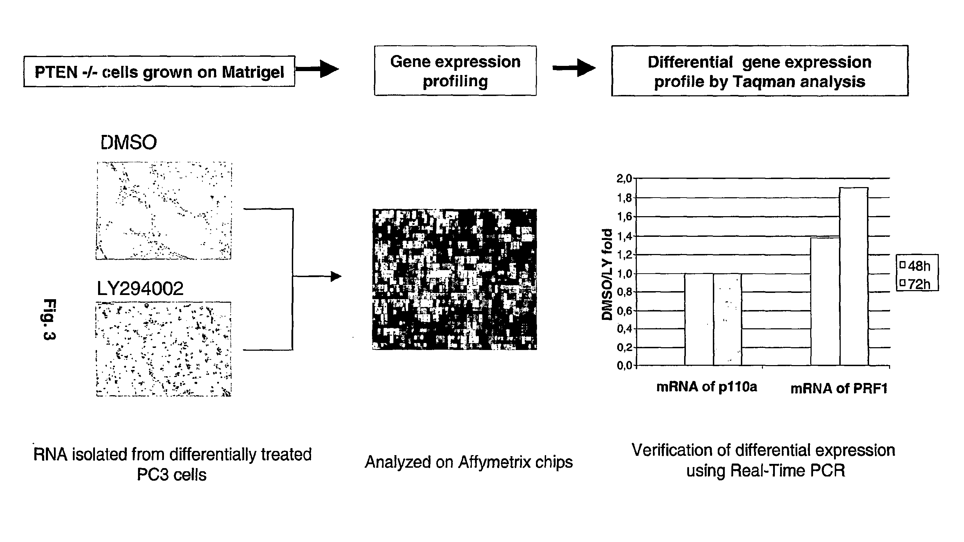

The basic experimental approach is shown in Fig. 3. PC3 cells grown on Matrigel were either treated with DMSO or the PI 3-K inhibitor LY294002 and total RNA were isolated from each sample. Differential Affymetrix gene expression profiling was performed and expression was confined using real time RT-PCR Taqman assay. p110α was used as a non-differential standard.

PC3 cells are PTEN -/- which means that the tumor suppressor PTEN is factually lacking in these cells so that the PI 3-kinase pathway is permanently activated which leads to an increased metastatic activity or behaviour of the cells which is expressed by their growth pattern in the matrigel assay. (Petersen, O.W., Ronnov-Jessen, L., Howlett, A.R. and Bissell, M.J. (1992) Interaction with basement membrane serves to rapidly distinguish growth and differentiation pattern of normal and malignant human breast epithelial cells. Proc Natl Acad Sci U S A, 89, 9064-9068. (Auch: Sternberger et al., 2002 Antisense & Nucleic acid drug development 12:131-143)

In connection therewith it is to be noted that the PC3 cells were grown on Matrigel and taken this as a model system which is close to the in vivo environment the RNA isolated therefrom, it is assumed to be closer to the in situ situation or results than any preparation obtained from cells grown in a non-matrigel environment such as a conventional cell culture plate.

Besides from PC-3 cells, other cells were grown and tested such as PNT-1A, MCF-10A and HELA. Various growth surfaces were used and the cells treated with compounds deemed or known to affect the PI 3-kinase pathway. The results are shown in the table depicted in Fig. 7. As may be taken from the readout expressed as "change" in the utmost two right columns, the signal obtained in the Affymetrix gene expression profiling was increased except for the following combinations of compounds administered to the cells, namely in case of PC-3 cells a combination of mismatch GBs directed against the catalytic subunits of PI 3-kinase p110a and p110b and the same mismatch in connection with LY (which is LY294002), in case of PNT-1A cells an antisense molecule designated as PTEN 17 and the respective mismatch, and in case of HELA cells the combination of Tam and DMSO after 48 h and 72 plus 96 h, respectively. Stable cell lines expressing an inducible form of the constitutively active PI 3-kinase, Mp110*ER, have been described (Klippel et al., 1998; Sternberger et al., 2002). Pools of stably transfected cells in growth medium were stimulated with 200 nM of the inducer 4-OHTamoxifen (Tam) in DMSO as described previously.

### Example 4: Screening for optimum antisense oligonucleotides directed to PRF1

In order to screen for optimum antisense oligonucleotides directed to PRF1 eight GeneBlocs were chosen over the total mRNA sequence of PRF1.

PC3 cells were transfected with different GeneBloc concentrations as described and mRNA levels were determined 24hrs post transfection using Taqman assays with 300nM of NM_019058 specific forward and reverse primer and 100nM probe and 40nM forward and reverse primer and 100nM probe for human β-action.

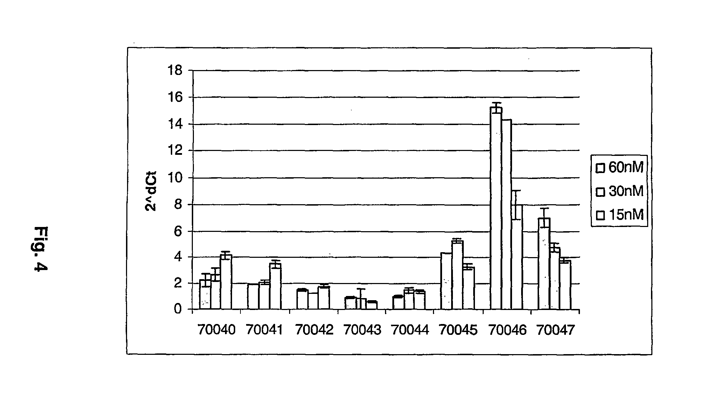

The results of this procedure which is also referred to herein as primary GeneBloc screen, is depicted in Fig. 4. From the results obtained GeneBlocs 70034 and 70044 were selected for further studies.

In connection with the GeneBloc as used herein in the various examples it is to be noted that they are all third generation antisense oligonucleotides as specified herein which means, as also obvious from table 1, that the upper case letters represent the deoxyribonucleotides which were linked through a phosphorothioate rather than a phosphordiester linkage.

**Table 1: Overview of the various GeneBlocs used, their alias, mismatches relative to the target nucleic acid and the sequences' structural characteristics**

| **GeneBloc No** | **Alias** | **MM** | **Sequence** | **SEQ ID NO.** |
|---|---|---|---|---|
| 70040 | FLJ:1558L21 | 0 | gctcaaCTCTGCAGTacacga | 4 |
| 70041 | FLJ:1356L21 | 0 | cttggtCCCTTCAGAccagta | 5 |
| 70042 | FLJ:1006L21 | 0 | cagtttTCCAACCACtggaat | 6 |
| 70043 | FLJ:954L21 | 0 | cccaaaAGTTCAGTCgtctct | 7 |
| 70044 | FLJ:975L21 | 0 | gctcctGCCTCTAGTctccac | 8 |
| 70045 | FLJ:470L21 | 0 | gtgttcATCCTCAGGgtcatc | 9 |
| 70046 | FLJ:1412L21 | 0 | ggtcagTAGTGATGCtccgat | 10 |
| 70047 | FLJ:571L21 | 0 | cttaccAACTGGCTAggcatc | 11 |
| 70168 | FLJ:954L21 | 4 | ccgaaaAGAACAGTGctctct | 12 |
| 70169 | FLJ:975L21 | 4 | gctcgtCCCTGTAGTgtccac | 13 |

In addition it is to be noted that any of the "t" above are actually "u" given the fact that the above antisense oligonucleotides are GeneBlocs, i. e. third generation antisense oligonucleotides.

Further antisense molecules as used in the various examples and embodiments of the present invention can be taken from table 2 indicating the names, i. e. internal references of the antisense molecules, their sequences and the sequences' characteristics. These antisense molecules are GeneBlocs, i. e. third generation antisense oligonucleotides. Underscores indicate mismatches relative to the target sequences.

**Table 2: Overview of further GeneBlocs used**

| | |
|---|---|
| PTEN 48 | guccuuuCCCAGCTTTacaguga |
| PTEN 52 | cuggaucAGAGTCAGTgguguca |
| PTEN 53 | ucuccuuTTGTTTCTGcuaacga |
| PTEN 57 | ugccacuGGTCTGTAAuccaggt |
| mm PTEN 52 | cuggaugAGACTGAGTgcuguca |
| mm PTEN 53 | ucucauuTTCTTTGTGcucacga |
| p110α 79 | acuccaaAGCCTCTTGcucaguu |
| p110α 82 | uaccacaCTGCTGAACcagucaa |
| p110β 88 | caaauucCAGTGGTTCauuccaa |
| p110β 93 | ggcuaacTTCATCTTCcuuccca |
| mm p110α 79 | acugcaaACCCTGTTGcucacuu |
| mm p110β 93 | ggcuaagTTCTTCATCcuugcca |
| PTEN 17 | cccuuuCCAGCTTTAcaguga |
| mm PTEN 17 | ccguuuGCACCTTTAgaguga |
| HIF1alpha 66 | gguaguGGTGGCATTagcagu |
| mm HIF1alpha 66 | gguagaGGTGCCAATugcagu |
| HIF1alpha 67 | ugacucCTTTTCCTGcucugu |
| mm HIF1alpha 67 | ugacucCTTTTCCTGcucugu |
| AKT1-GB | gucuugATGTACTCCccucgu |
| mm-AKT1 | guguugATCTAGTCCccuccu |
| AKT2-GB | uccuugTACCCAATGaaggag |
| mm-AKT2 | ucguugTAGCCAATCaacgag |

### Example 5: Selective knockdown of PRF1

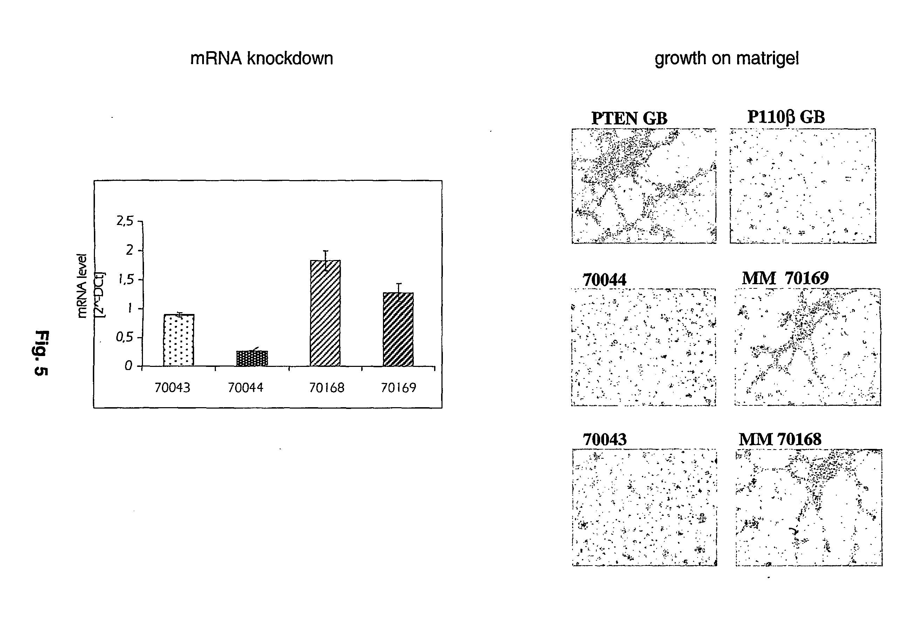

In order to prove that PRF1 is a suitable downstream drug target of the PI 3-kinase pathway the two particularly advantageous GeneBlocs as obtained from example 4, i. e. 70044 and 70043, were used in a matrigel based growth experiment. The matrigel growth experiment is taken as a surrogate model which shows the metastasis and migration behavior of the respective cell. A more confluent growth of the cells is taken as an indication that their metastasis and migration behavior is increased which allows the cells to spread over the three-dimensional structure provided by the matrigel. The result of this example is depicted in Fig. 5.

PC3 cells were transfectd and seeded on matrigel as described and growth was monitored. mRNA was isolated from an aliquot of the cells seeded on matrigel and analysed using Taqman assay (left panel). PRF1 (NM_019058) specific mRNA was standardized to internal p110α mRNA levels. A PTEN specific GeneBloc is used as a negative control in the PTEN^{-/-}PC-3 cells and a p110β specific GeneBloc is used as a positive control for growth in extracellular matrix. Specific growth inhibition is shown by comparing growth of cells treated with PRF1 specific GeneBlocs 70043 and 70044, respectively, versus their corresponding mismatched oligonucleotides 70168 and 70169, respectively.

From Fig. 5A and Fig. 5B it may be taken that the two preferred GeneBlocs, namely 70043 and 70044 result in a significant knockdown of the mRNA of PRF1 whereby this result corresponds to the ones obtained from the matrigel growth assay. The y axis represents the amount of mRNA as determined by the DCt method in accordance with the instructions of Applied Biosystems.

### Example 6: Generation and specificity of a poylclonal antibody against PRF1

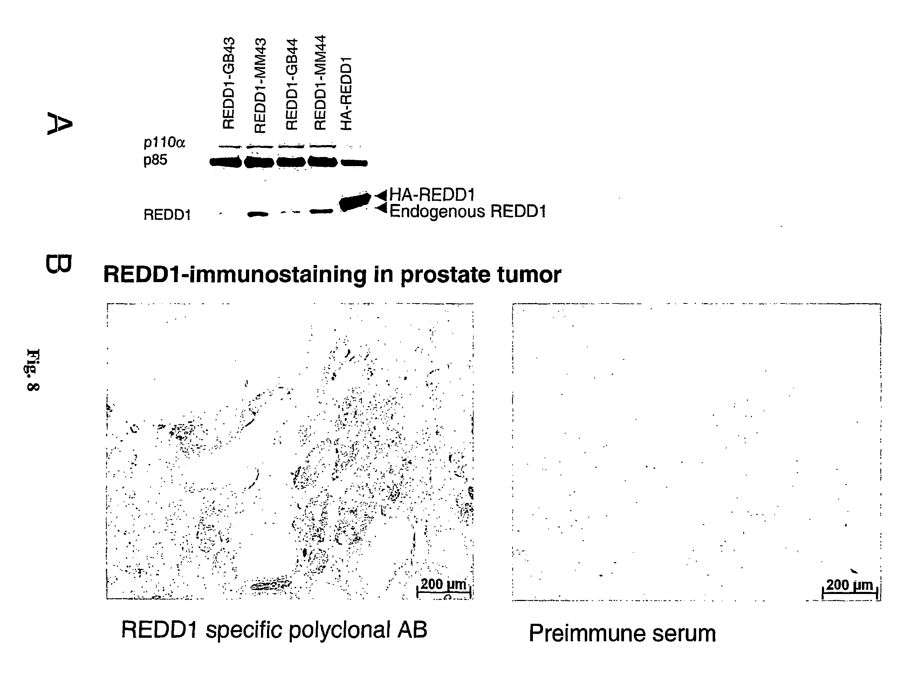

A polyclonal antibody was generated against PRF1 using standard techniques. The polyclonal antibody thus obtained was tested for its specificity for PRF1. For that purpose, cell lysates from PC-3 cells treated with PRF1 specific antisense molecules, more particularly gene blocks, or mismatches thereof and cells transfected with expression plasmides for recombinant HA-tagged PRF1 were prepared. The lysates were analysed by Western blot analysis using the polyclonal antibody and the results are depicted in Fig. 8A.

As may be taken from Fig. 8A PC-3 cells treated with gene block number 70043 (GB43, see table 1) which is specific to the nucleic acid sequence as disclosed herein shows a clear reduction in expressed PRF1-protein whereby PI 3-kinase still being extensively expressed as illustrated by p110α and p85 specific antibodies. A second antisense molecule, namely GB44, also designed against PRF1, also led to a significant decrease in the expression of the PRF1 protein. Again, the mismatch molecule as in case of the mismatch to GB43, MM43, PRF1 protein was significantly expressed in the cells. The lysates from cells transfected with expression plasmids for recombinant HA-tagged PRF1 show two bands, one for the HA-tagged PRF1 and the other one for the endogenous PRF1.

These results confirm that the polyclonal antibody exhibits a specificity for PRF1 and is thus a valuable tool in monitoring the presence and absence, respectively, of PRF1.

### Example 7: Immunostaining for PRF1 in prostate tumor tissue

The antibody the generation of which and characterisation of which is described in example 6 was used for staining human prostate tumor tissue. Using the polyclonal serum in a 1:1000 dilution the expression of PRF1 at the protein level can be clearly visualized as depicted in Fig. 8 B, whereby the left photograph depicts the staining using the polyclonal antibody described in example 6, whereas the right photograph depicts a slice of the prostate tumor stain using preimmune serum.

### Example 8: PRF1 protein expression depends on various members of the PI 3-kinase pathway

In order to demonstrate that PRF1 is a downstream target of the PI 3-kinase pathway and the HIF1α pathway, various experiments were carried out, whereby compounds were used known to be specific to various elements of the respective pathways and the impact of such compounds on the PRF1 protein level investigated.

### a) Impact of PI 3-kinase activity on PRF1 protein expression

To investigate whether PI 3-kinase activity has an impact on PRF1 protein expression, PC-3 cells were treated either with LY294002 or with DMSO as control. Analysis was performed by Western Blot analysis using the polyclonal antibody as detection means for PRF1 protein. Apart from PRF1 protein, also expression of Akt and phosphorylated Akt (P* Akt) was monitored using respective specific antibodies. Additionally, the expression of p110α and p85 was monitored.

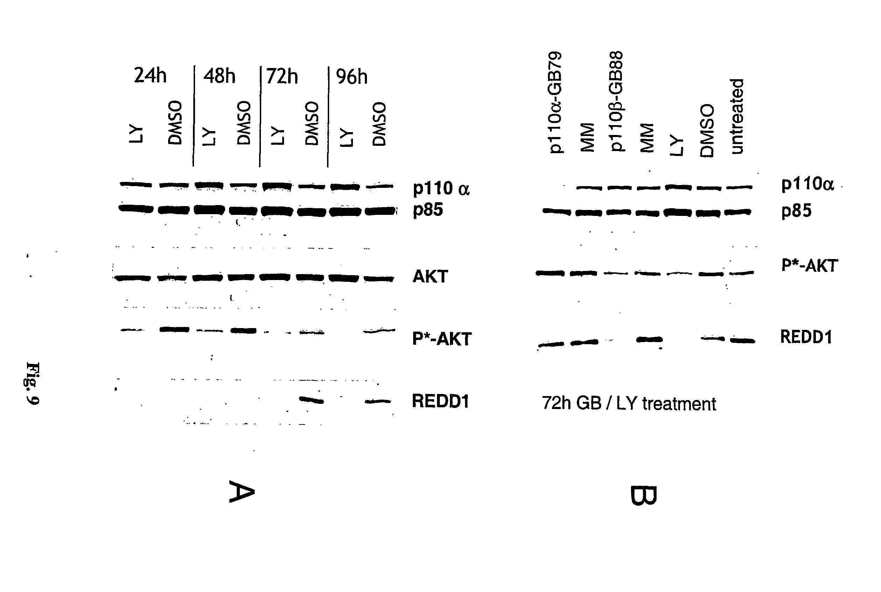

The results are depicted in Fig. 9A.

After 72 and 96 h, respectively, PRF1-protein could only be detected in cells treated with DMSO. Under the influence of LY294002 the cascade consisting of Akt and phosphorylated Akt, respectively, and PRF1 protein was down regulated.

Using the same markers, the cells were now treated with an antisense molecule, namely a GeneBloc (GB) designated p110β-GB 88 to specifically inhibit PI 3-kinase.

The results are shown in Fig. 9B.

As may be taken from the third and the fifth lane, the GeneBloc against the catalytically active subunit p110β had a similar effect as the known PI 3-K inhibitor LY294002 confirming that PRF1 is a downstream target the protein expression of which is influenced by PI 3-kinase. An antisense molecule directed against p110α or a mismatch antisense molecule or DMSO did not have any effect on the expression of PRF1 at the protein level.

### b) Impact of Akt and HIF1α on PRF1 protein expression

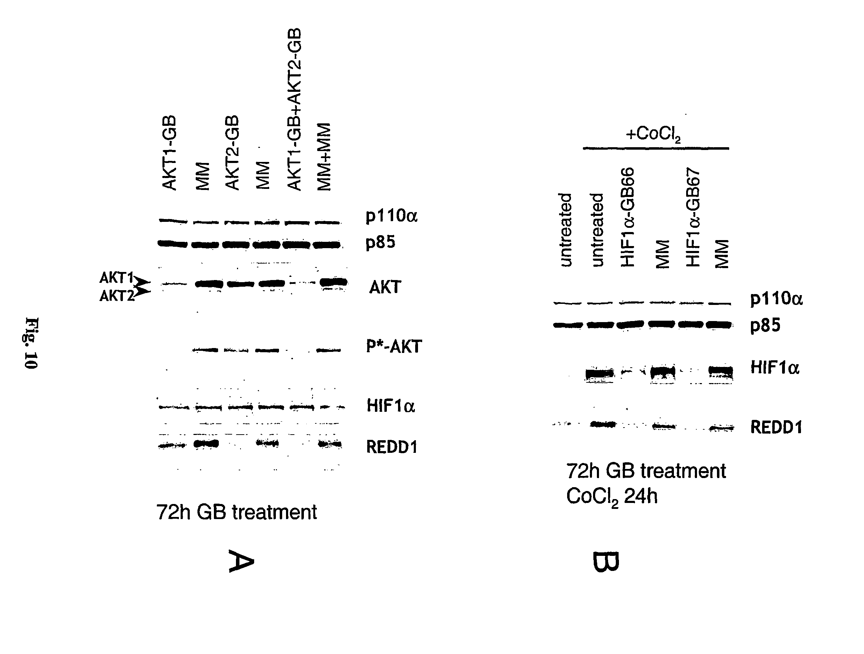

To investigate whether the expression of PRF1 at the protein level would also be dependent on Akt (PKB) activity and HIF1α activity PC-3 cells were treated with Akt specific antisense molecules. More particularly, two antisense molecules, designated Akt1-GB and Akt2-molecule were used with the readout being the same as discussed in connection with Fig. 9, whereby, additionally, the HIF1α protein expression was monitored using a commercially available protein G-purified polyclonal antibody (R&DSystems, Lot Number: IUGO13071). The result is shown in Fig. 10A. As may be taken from Fig. 10A 72 h after treatment with the antisense molecule cells treated with Akt2 GeneBloc and those cells treated with Akt1 GeneBloc together with Akt2 GeneBloc showed a reduction in the PRF1 protein level indicating that Akt1 and Akt2, respectively, exhibit a certain inhibitory effect on the level of PRF1 protein expression.

The same experiment using the same markers for monitoring except Akt and P*-Akt was carried out with cells grown under hypoxic conditions which was realized by treating the cells with CoCl₂. Two different forms of antisense molecules, namely HIF1α-GB66 and HIF1α-GB67 were used and respective mismatches used as controls. The GeneBlocs significantly reduced the expression of HIF1α at the protein level which in turn reduced the expression of PRF1 protein. This result was obtained after 72 h treatment with GeneBlocs and CoCl₂ treatment for 24 h.

From this result it can be taken that the protein level of PRF1 is both dependent on the activity of Akt and HIF1α, although the impact of HIF1α on the expression of PRF1 at the protein level seems to be more pronounced than the one of Akt.

These results confirm that PRF1 is a downstream effector of the PI 3-kinase and the HIF1α signalling. The presented data on pathway defection suggests that PRF1 is dependent on HIF1α activity under hypoxic growth conditions. In addition there seems to be an additional Akt dependency of PRF1 expression which seems to be not HIF1α dependent. This regulatory relationship is illustrated in Fig. 12.

### Example 9: Phenotypic analysis of cells having HIF1alpha protein and PRF1 protein inhibited

The purpose of the experiment underlying this example was to investigate the phenotypic changes of cells showing an inhibition of the expression of HIF1 alpha and PRF1, respectively, at the protein level.

Cells were grown on a matrigel surface and treated with the antisense molecules (geneblocs) and respective controls and under the conditions described in the above experiments.

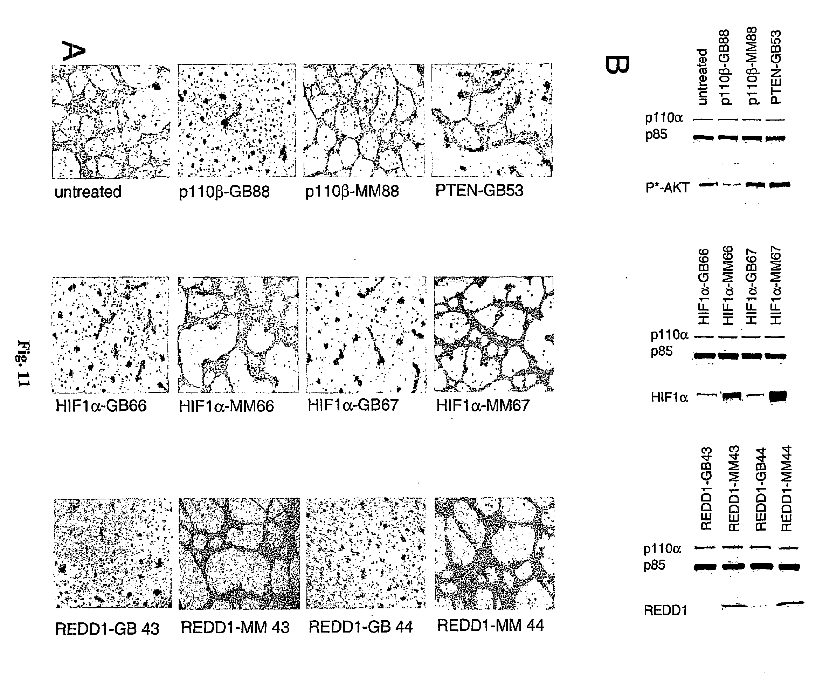

The results are depicted in Fig. 11A and Fig. 11B, whereby each row consists of a part of Fig. 11A and Fig. 11B which will be discussed together.

Fig. 11A shows a sequence of photographs of cells grown on matrigel surface and treated with the respective antisense molecules, whereas Fig. 11B shows the result of a Western Blot analysis performed on cells which were obtained from the matrigel surface. A lane of the Blot represents the cell lysate of the cells shown in the corresponding photograph. As a read-out, p110 alpha and p85 are shown as loading control and the component of the PI-3K pathway investigated, i.e. AKT, monitored as P*AKT, HIF1 alpha and PRF1.

Untreated cells show the growth pattern of normal tumor cells in the matrigel assay. A similar phenotype is observed when a mismatch of the antisense molecule directed against p110β is used. Using an anti-sense molecule directed against PTEN which in turn is inhibiting PI-3K, shows the phenotype of a cell line not effected by this tumor repressor (Fig. 11A, first row). As expected P*-AKT is significantly reduced under these condition and represents the read-out for functional p110β knock-down (Fig. 11B, first row). A similar phenotype due to loss of function as with the knock-down of p110β is observed when using anti-sense molecules against HIF1alpha with the mismatches not leading to a change in the phenotype (Fig. 11A, second row). The Western Blot analysis confirmed this and indicates a reduced protein level of HIF1alpha for both HIF1alpha specific antisense molecules (Fig. 11B, second row). Finally, using PRF1 specific anti-sense molecules again the same loss of function phenotype can be observed (picture 1 and 3 of the third row of Fig. 11A) going along with a decreased expression of PRF1 protein also in the Western Blot analysis Fig. 11B, third row).

To summarize, these results confirm that PRF1 is also at the protein level a downstream, effector of the PI 3-kinase signalling. Also, PFR1 is dependent on HIF1alpha activity under hypoxic growth conditions. In addition, there seems to be an additional AKT dependence of PRF1 expression which seems to be not HIF1alpha dependent.

### Example 10: Inhibition of prostatic tumor growth by specific RNA interference

This experiment is an example of a successful design of small interfering RNA (siRNA) which allows that the downstream drug target PRF1 is specifically addressed. siRNA molecules were generated by promoter (u6+2) driven expression of target specific sequences.

### Construction of siRNA expression plasmids

The pol III promoter cassettes U6+2 were PCR generated using synthetical oligonucleotides and cloned into an *Eco*RI/*Xho*I restriction site of a pUC-derived vector. The specific siRNA insert was cloned using a nonpalindromic restriction enzyme (*Bsm*BI with 5'overhang TTTT, 3'overhang GGCA). Inserts were generated by annealing two synthetic oligonucleotides with 5'CCGT and 3'AAAA overhangs. The expression cassette comprising the promoter, the siRNA to be expressed and a terminator sequence reads in case of PRF1 specific siRNA as follows:

U6+2 synthetic promoter and PRF1 specific siRNA in bold, (EcoRI; XhoI)

This expression cassette may, in principle, be cloned into any expression vector.

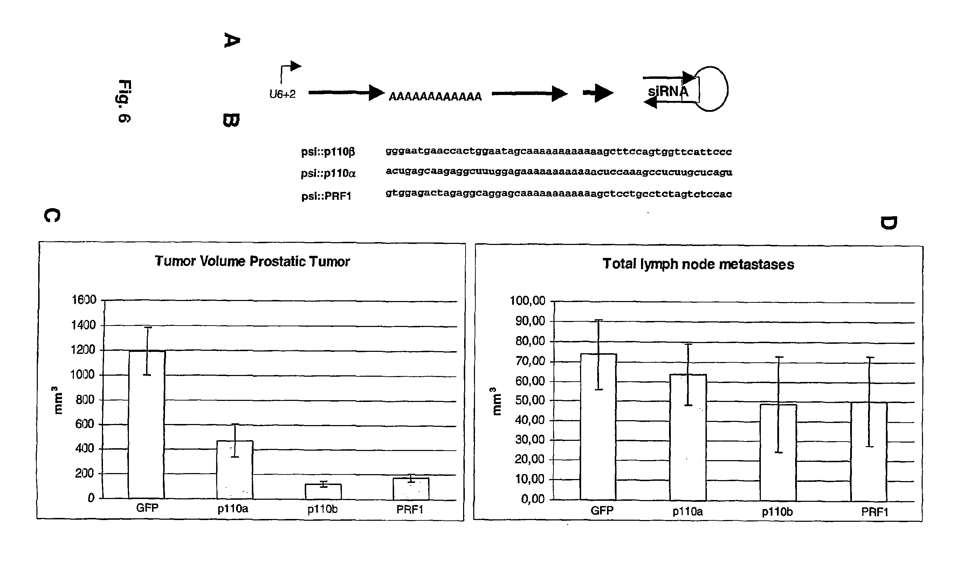

The basic design of the siRNA expressing construct is also depicted in Fig. 6A from which it may be taken that the siRNAs are such designed as to form an intracellular loop which is generated by the poly-A-stretch. Fig. 6B shows the various siRNA constructs used in the present example, namely for p110 beta (SEQ ID NO. 14), p110 alpha (SEQ ID NO. 15) and for the mRNA of PRF1.

The various siRNA constructs such as the ones directed to p 110 alpha, p110 beta and PTEN as well as the PRF1 specific siRNA were cloned in the same vector construct. PC-3 cells (1x10⁶ cells) were transfected with 6 µg of the respective siRNA expression plasmids or a EGFP expression plasmid *using Effectene^{™} (Qiagen, Hilden, Germany) according to the manufacturer's instruction,* expressing siRNA s to p110 α, p110β, or PRF1 or expressing full-length EGFP which served as control for tumor growth of stably transfected PC-3 cells were transfected. 48 hours post transfection cells were trypsinized and diluted and reseeded in 150 mm dishes containing 500 µg/ml Geneticin. Medium with 500 µg/ml Geneticin was replaced daily and 7 days post transfection replaced by medium containing 600 µg/ml Geneticin. Resistent pools of cells were expanded and harvested, cell number determined and prepared for animal experiment as described.

The results are shown in Fig. 6, whereby more particularly in Fig. 6C the volume of primary prostate tumors in mm³ 56 days post inoculation are shown and in Fig. 6D the volumes of total lymph node metastasis in mm³ 56 days post inoculation are shown.

It may be taken from said figures that prostatic tumor growth can be significantly inhibited by administering a PRF1 specific siRNA. The extent of inhibition is thereby similar to a siRNA construct directed to p110 beta. However, taken the position of p110 beta and PRF1, respectively, in the PI 3-kinase pathway, it is to be understood that the siRNA specific to PRF1 allows a more specific addressing and thus modulation of a part or branch of the PI 3-kinase pathway and processes linked thereto compared to addressing p110 beta. The same is also true in case total lymph node metastasis is taken into consideration whereby the effects are less prominent there.

## Claims

1. In vitro use of a polypeptide factor or a fragment thereof, whereby the fragment has the effects of the polypeptide factor in terms of wound healing, tumor growth, tumorigenesis, metastasis, cell migration, cell motility in extracellular matrix and cell growth in extracellular matrix , whereby the factor
a) comprises an amino acid sequence according to SEQ ID NO. 1;
b) has an amino acid sequence according to data bank entries gi 9506687 or NP_061931. 1;
c) is encoded by a nucleic acid, whereby the
ca) nucleic acid comprises
i) a nucleic acid according to SEQ ID NO. 2 or SEQ ID NO. 3; or
ii) a nucleic acid sequence according to data bank entries gi 9506686 or NM_019058.1;
cb) the nucleic acid codes for the factor as of a) or b) and whereby the nucleic acid would hybridise but for the degeneracy of the genetic code, to a nucleic acid according to ca); or
cc) the nucleic acid codes for the factor as of a) or b) and whereby the nucleic acid hybridises under stringent conditions to a nucleic acid which is complementary to the nucleic acid according to ca);
as a downstream target or a downstream marker of the PI 3-kinase pathway, preferably as a downstream drug target of the PI 3-kinase pathway, in drug screening, drug design and drug development, whereby the drug is useful for wound healing, inhibiting tumor growth, tumorigenesis, metastasis, cell migration, cell motility in extracellular matrix and cell growth in extracellular matrix.

2. In vitro use of a polypeptide factor, whereby the factor
a) comprises an amino acid sequence according to SEQ ID NO. 1;
b) has an amino acid sequence according to data bank entries gi 9506687 or NP_061931.1;
c) is encoded by a nucleic acid, whereby the
ca) nucleic acid comprises
i) a nucleic acid according to SEQ ID NO. 2 or SEQ ID NO. 3; or
ii) a nucleic acid sequence according to data bank entries gi 9506686 or NM_019058. 1;
cb) the nucleic acid codes for the factor as of a) or b) and whereby the nucleic acid would hybridise but for the degeneracy of the genetic code, to a nucleic acid according to ca); or
cc) the nucleic acid codes for the factor as of a) or b) and whereby the nucleic acid hybridises under stringent conditions to a nucleic acid which is complementary to the nucleic acid according to ca);
as a marker for a process, whereby the process is a PI 3-kinase pathway regulated process, whereby the process is selected from the group consisting of wound healing, metastasis, cell migration, cell motility in extracellular matrix and cell growth in extracellular matrix.

3. Use according to claim 2, whereby the factor is a marker for transformed cells, preferably for invasive cells.

4. Use of a polypeptide factor or a fragment thereof, whereby the fragment has the effects of the polypeptide factor in terms of wound healing, tumor growth, tumorigenesis, metastasis, cell migration, cell motility in extracellular matrix and cell growth in extracellular matrix, whereby the factor
a) comprises an amino acid sequence according to SEQ ID NO. 1;
b) has an amino acid sequence according to data bank entries gi 9506687 or NP_061931.1;
c) is encoded by a nucleic acid, whereby the
ca) nucleic acid comprises
i) a nucleic acid according to SEQ ID NO. 2 or SEQ ID NO. 3; or
ii) a nucleic acid sequence according to data bank entries gi 9506686 or NM_019058.1;
cb) the nucleic acid codes for the factor as of a) or b) and whereby the nucleic acid would hybridise but for the degeneracy of the genetic code, to a nucleic acid according to ca); or
cc) the nucleic acid codes for the factor as of a) or b) and whereby the nucleic acid hybridises under stringent conditions to a nucleic acid which is complementary to the nucleic acid according to ca);
for the manufacture of a medicament for the treatment and/or prevention of a disease,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies,
or whereby the medicament is for wound healing.

5. Use of a polypeptide factor or a fragment thereof, whereby the fragment has the effects of the polypeptide factor in terms of wound healing, tumor growth, tumorigenesis, metastasis, cell migration, cell motility in extracellular matrix and cell growth in extracellular matrix, whereby the factor
a) comprises an amino acid sequence according to SEQ ID NO. 1;
b) has an amino acid sequence according to data bank entries gi 9506687 or NP_061931.1;
c) is encoded by a nucleic acid, whereby the
ca) nucleic acid comprises
i) a nucleic acid according to SEQ ID NO. 2 or SEQ ID NO. 3; or
ii) a nucleic acid sequence according to data bank entries gi 9506686 or NM_019058.1;
cb) the nucleic acid codes for the factor as of a) or b) and whereby the nucleic acid would hybridise but for the degeneracy of the genetic code, to a nucleic acid according to ca); or
cc) the nucleic acid codes for the factor as of a) or b) and whereby the nucleic acid hybridises under stringent conditions to a nucleic acid which is complementary to the nucleic acid according to ca);
for the manufacture of a diagnostic agent for the diagnosis of a disease,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies,
or whereby the diagnostic agent is for wound healing.

6. Use of a nucleic acid, whereby such nucleic acid is
a) a nucleic acid coding for a factor or a fragment thereof, whereby the factor is
i) a polypeptide comprising an amino acid according to SEQ ID NO. 1; or
ii) a polypeptide having a sequence according to data bank entries gi 9506687 or NP_061931.1;
b) a nucleic acid coding for the factor as of a), whereby the nucleic acid comprises
i) a nucleic acid according to SEQ ID NO. 2 or SEQ ID NO. 3; or
ii) a nucleic acid sequence according to data bank entries gi 9506686 or NM_019058.1;
c) a nucleic acid coding for the factor as of a), whereby the nucleic acid would hybridise, but for the degeneracy of the genetic code, to a nucleic acid according to b); or
d) a nucleic acid coding for the factor as of a), whereby the nucleic acid hybridises under stringent conditions to a nucleic acid which is complementary to the nucleic acid according to b);
for the manufacture of a medicament for the treatment and/or prevention of a disease, whereby the fragment has the effects of the polypeptide factor in terms of wound healing, tumor growth, tumorigenesis, metastasis, cell migration, cell motility in extracellular matrix and cell growth in extracellular matrix,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), gastrointestinal hamartomas, lipomas, thyroid adenomas, fibrocystic disease of the breast, cerebellar dysplastic gangliocytoma and breast and thyroid malignancies.
or whereby the medicament is for wound healing.

7. Use of a nucleic acid, whereby such nucleic acid is
a) a nucleic acid coding for a factor or a fragment thereof, whereby the factor is
i) a polypeptide comprising an amino acid according to SEQ ID NO. 1; or
ii) a polypeptide having a sequence according to data bank entries gi 9506687 or NP_061931.1;
b) a nucleic acid coding for the factor as of a), whereby the nucleic acid comprises
i) a nucleic acid according to SEQ ID NO. 2 or SEQ ID NO. 3; or
ii) a nucleic acid sequence according to data bank entries gi 9506686 or NM_019058.1;
c) a nucleic acid coding for the factor as of a), whereby the nucleic acid would hybridise, but for the degeneracy of the genetic code, to a nucleic acid according to b); or
d) a nucleic acid coding for the factor as of a), whereby the nucleic acid hybridises under stringent conditions to a nucleic acid which is complementary to the nucleic acid according to b);
thereof for the manufacture of a diagnostic agent for the diagnosis of a disease, whereby the fragment has the effects of the polypeptide factor in terms of wound healing, tumor growth, tumorigenesis, metastasis, cell migration, cell motility in extracellular matrix and cell growth in extracellular matrix,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), mucocutaneous lesions, such as trichilemmomas, gastrointestinal hamartomas, lipomas, thyroid adenomas, fibrocystic disease of the breast, cerebellar dysplastic gangliocytoma and breast and thyroid malignancies
or whereby the diagnostic agent is for wound healing.

8. Use according to any of claims 1 and 3 to 7, whereby
the disease is **characterized in that** the cells being involved in said disease lack PTEN activity, or show a hyperactivation of the PI 3-kinase pathway, or show an increased aggressive behaviour, or are tumor cell, preferably cells of a late stage tumor.

9. In vitro use of a polypeptide factor or a fragment thereof, whereby the factor
a) comprises an amino acid sequence according to SEQ ID NO. 1;
b) has an amino acid sequence according to data bank entries gi 9506687 or NP_061931.1;
c) is encoded by a nucleic acid, whereby the
ca) nucleic acid comprises
i) a nucleic acid according to SEQ ID NO. 2 or SEQ ID NO. 3; or
ii) a nucleic acid sequence according to data bank entries gi 9506686 or NM_019058.1;
cb) the nucleic acid codes for the factor as of a) or b) and whereby the nucleic acid would hybridise but for the degeneracy of the genetic code, to a nucleic acid according to ca); or
cc) the nucleic acid codes for the factor as of a) or b and whereby the nucleic acid hybridises under stringent conditions to a nucleic acid which is complementary to the nucleic acid according to ca);
as a target molecule for the development of a medicament for the treatment and/or prevention of a disease, whereby the fragment has the effects of the polypeptide factor in terms of wound healing, tumor growth, tumorigenesis, metastasis, cell migration, cell motility in extracellular matrix and cell growth in extracellular matrix,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer prostate cancer, Bannayan-Zonana syndrome, LDD (Lhermitte-Duclos' syndrome) hamartoma-macrocephaly diseases including Cow disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies,
or whereby the medicament is for wound healing.

10. In vitro use of a polypeptide factor or a fragment thereof, whereby the factor
a) comprises an amino acid sequence according to SEQ ID NO. 1;
b) has an amino acid sequence according to data bank entries gi 9506687 or NP_061931.1;
c) is encoded by a nucleic acid, whereby the
ca) nucleic acid comprises
i) a nucleic acid according to SEQ ID NO. 2 or SEQ ID NO. 3; or
ii) a nucleic acid sequence according to data bank entries gi 9506686 or NM_019058.1;
cb) the nucleic acid codes for the factor as of a) or b) and whereby the nucleic acid would hybridise but for the degeneracy of the genetic code, to a nucleic acid according to ca); or
cc) the nucleic acid codes for the factor as of a) or b) and whereby the nucleic acid hybridises under stringent conditions to a nucleic acid which is complementary to the nucleic acid according to ca);
as a target molecule for the development of a diagnostic agent for the diagnosis of a disease, whereby the fragment or derivative has the effects of the polypeptide factor in terms of wound healing, tumor growth, tumorigenesis, metastasis, cell migration, cell motility in extracellular matrix and cell growth in extracellular matrix,
whereby the disease is selected from the group consisting of late stage tumor, metastatic cancer, and any pathological conditions involving the PI 3-kinase pathway,
whereby such conditions are selected from the groups consisting of colorectal carcinomas, gliomas, endometrial cancers, adenocarcinomas, endometrial hyperplasias, Cowden's syndrome, breast-ovarian cancer, prostate cancer; Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR) trichilemmomas, gastrointestinal hamartomas, lipomas, thyroid adenomas, fibrocystic disease of the breast, cerebellar dysplastic gangliocytoma and breast and thyroid malignancies,
or whereby the diagnostic agent is for wound healing.

11. In vitro use of a nucleic acid, whereby said nucleic acid is
a) a nucleic acid coding for a factor or a fragment thereof, whereby the factor is
i) a polypeptide comprising an amino acid according to SEQ ID NO. 1; or
ii) a polypeptide having a sequence according to data bank entries gi 9506687 or NP_061931.1;
b) a nucleic acid coding for the a factor as of a), whereby the nucleic acid comprises
i) a nucleic acid according to SEQ ID NO. 2 or SEQ ID NO. 3; or
ii) a nucleic acid sequence according to data bank entries gi 9506686 or NM_019058.1;
c) a nucleic acid coding for the factor as of a), whereby the nucleic acid would hybridise, but for the degeneracy of the genetic code, to a nucleic acid according to b); or
d) a nucleic acid coding for the factor as of a), whereby the nucleic acid hybridises under stringent conditions to a nucleic acid which is complementary to the nucleic acid according to b);
as a target molecule for the development of a medicament for the treatment and/or prevention of a disease, whereby the fragment has the effects of the polypeptide factor in terms of wound healing, tumor growth, tumorigenesis, metastasis, cell migration, cell motility in extracellular matrix and cell growth in extracellular matrix,
whereby the disease is selected from the group consisting of late stage tumor, metastatic cancer, and any pathological conditions involving the PI 3-kinase pathway,
whereby such conditions are selected from the group consisting of colorectal, carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), as trichilemmomas, gastrointestinal hamartomas, lipomas, thyroid adenomas, fibrocystic disease of the breast, cerebellar dysplastic gangliocytoma and breast and thyroid malignancies,
or whereby the medicament is for wound healing.

12. In vitro use of a nucleic acid, whereby said nucleic acid is
a) a nucleic acid coding for a factor or a fragment thereof, whereby the factor is
i) a polypeptide comprising an amino acid according to SEQ ID NO. 1; or
ii) a polypeptide having a sequence according to data bank entries gi 9506687 or NP_061931.1;
b) a nucleic acid coding for the factor as of a), whereby the nucleic acid comprises
i) a nucleic acid according to SEQ ID NO. 2 or SEQ ID NO. 3; or
ii) a nucleic acid sequence according to data bank entries gi 9506686 or NM_019058.1;
c) a nucleic acid coding for the factor as of a), whereby the nucleic acid would hybridise, but for the degeneracy of the genetic code, to a nucleic acid according to b); or
d) a nucleic acid coding for the factor as of a), whereby the nucleic acid hybridises under stringent conditions to a nucleic acid which is complementary to the nucleic acid according to b);
as a target molecule for the development of a diagnostic agent for the diagnosis of a disease, whereby the fragment has the effects of the polypeptide factor in terms of wound healing, tumor growth, tumorigenesis, metastasis, cell migration, cell motility in extracellular matrix and cell growth in extracellular matrix, whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LID (Lhermitte-Duclos' syndrome) hamartoma-macrocephaly diseases including Cowden's disease (CD) and Bannayall-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies,
or whereby the diagnostic agent is for wound healing.

13. The use according to any of claims 9 to 12, **characterised in that** the medicament and/or the diagnostic agent comprises an agent, which is selected from the group consisting of antibodies, high affinity binding peptides, anticalines, antisense molecules, aptameres, spiegelmers and RNAi molecules.

14. The use according to claim 13, **characterised in that** the agent interacts with a factor or a fragment thereof as defined in claim 1.

15. The use according to claim 13, **characterised in that** the agent interacts with the nucleic acid as defined in claim 6, in particular with mRNA, genomic nucleic acid or cDNA coding for the polypeptide factor as defined in claim 1.

16. Use of an antibody or part thereof which interacts with the polypeptide factor or the fragment thereof as defined in claim1, for the development or manufacture of a medicament for the treatment and/or prevention of a disease and/or for the manufacture of a diagnostic agent for the diagnosis of a disease,
whereby the disease is selected from the group consisting of late stage tumor, metastatic cancer, and any pathological conditions involving the PI 3-kinase pathway, whereby such conditions are selected from the group consisting of
colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies
or whereby the medicament and/or the diagnostic agent is for wound healing.

17. Use of an aptamer or spiegelmer which interacts with the polypeptide factor or fragment thereof as defined in claim1, for the development or manufacture of a diagnostic agent for the diagnosis of a disease,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies
or whereby the diagnostic agent is for wound healing.

18. Use of a nucleic acid which interacts with a nucleic acid coding for the polypeptide factor or fragment thereof as defined in claim 1, for the development or manufacture of a medicament for the treatment and/or prevention of a disease and/or for the manufacture of a diagnostic agent for the diagnosis of a disease,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), as trichilemmomas, gastrointestinal hamartomas, lipomas, thyroid adenomas, fibrocystic disease of the breast, cerebellar dysplastic gangliocytoma and breast and thyroid malignancies
or whereby the medicament and/or the diagnostic agent is for wound healing,
whereby the interacting nucleic acid is an antisense oligonucleotide, a ribozyme and/or siRNA.

19. The use according to claim 18, **characterised in that** the nucleic acid coding for the polypeptide factor or a part thereof is the cDNA, mRNA or hnRNA.

20. Use of a kit for the characterisation of a disease or a condition which is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies, and wound healing,
whereby the kit comprises at least one agent which is selected from the group consisting of
a) the polypeptide factor or fragment thereof as defined in claim 1,
b) antibodies or part thereof specific for the polypeptide factor or the fragment thereof as of a),
c) a nucleic acid coding for a factor, whereby the factor is
i) a polypeptide comprising an amino acid according to SEQ ID NO. 1; or
ii) a polypeptide having a sequence according to data bank entries gi 9506687 or NP_061931.1;
d) a nucleic acid coding for the factor or fragment thereof as of a), whereby the nucleic acid comprises
i) a nucleic acid according to SEQ ID NO. 2 or SEQ ID NO. 3; or
ii) a nucleic acid sequence according to data bank entries gi 9506686 or NM_019058.1;
e) a nucleic acid coding for the factor or the fragment as of a), whereby the nucleic acid would hybridise but for the degeneracy of the genetic code, to a nucleic acid according to d); or
f) a nucleic acid coding for the factor or the fragment thereof as of a), whereby the nucleic acid hybridises under stringent conditions to a nucleic acid which is complementary to the nucleic acid according to d);
g) nucleic acids interacting with said polypeptide factor or a thereof, whereby the fragment has the effects of the polypeptide factor in terms of wound healing, tumor growth, tumorigenesis, metastasis, cell migration, cell motility in extracellular matrix and cell growth in extracellular matrix, and whereby the nucleic acids are selected from the group consisting of aptamers and spiegelmers, and
h) nucleic acids interacting with
ha) a nucleic acid coding for a factor, whereby the factor is
i) a polypeptide comprising an amino acid according to SEQ ID NO. 1; or
ii) a polypeptide having a sequence according to data bank entries gi 9506687 or NP_061931;
hb) a nucleic acid coding for a factor, whereby the nucleic acid comprises
i) a nucleic acid according to SEQ ID NO. 2 or SEQ ID NO. 3; or
ii) a nucleic acid sequence according to data bank entries gi 9506686 or NM_019058,
hc) a nucleic acid coding for the factor or a fragment thereof as of a), whereby the nucleic acid would but hybridise for the degeneracy of the genetic code, to a nucleic acid according to d); or
hd) a nucleic acid coding for the factor or a fragment thereof as of a), whereby the nucleic acid hybridises under stringent conditions to a nucleic acid which is complementary to the nucleic acid according to d);
and optionally at least one other compound.

21. Use according to any of claims 1 to 20, whereby the polypeptide factor is involved in a biological process, whereby the process is a PI 3-kinase pathway regulated process.

22. The use according to claim 21, whereby the process is a process selected from the group consisting of wound healing, metastasis, tumorigenesis, cell migration, cell motility in extracellular matrix and cell growth in extracellular matrix.

23. A method for the screening of an agent for the manufacture of a medicament for the treatment and/or prevention of a disease and/or for the screening of an agent for the manufacture of a diagnostic agent for the diagnosis of a disease,
whereby the disease is selected from the group consisting of late stage tumor, metastatic cancer, and any pathological conditions involving the PI 3-kinase pathway,
whereby such conditions are selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies,
or whereby the medicament and/or the diagnostic agent is for wound healing,
comprising the following steps:
a) providing a candidate compound,
b) providing an expression system for a polypeptide factor or a fragment thereof whereby the fragment has the effects of the polypeptide factor in terms of wound healing tumor growth, tumorigenesis, metastasis, cell migration, cell motility in extracellular matrix and cell growth in extracellular matrix, whereby the factor
ba) comprises an amino acid sequence according to SEQ ID NO.1;
bb) has an amino acid sequence according to data bank entries gi 9506687 or NP_061931.1;
bc) is encoded by a nucleic acid, whereby the
bca) nucleic acid comprises
i) a nucleic acid according to SEQ ID NO. 2 or SEQ ID NO. 3; or
ii) a nucleic acid sequence according to data bank entries gi 9506686 or NM_019058.1;
bcb) the nucleic acid codes for the factor or fragment thereof as of b), ba) or bb) and whereby the nucleic acid would hybridise but for the degeneracy of the genetic code, to a nucleic acid according to bca); or
bcc) a nucleic acid codes for the factor or fragment thereof as of, b), ba) or bb) and whereby the nucleic acid hybridises under stringent conditions to a nucleic acid which is complementary to the nucleic acid according to bca);
and/or a system, preferably an activity system, detecting the activity of said polypeptide factor;
c) contacting of the candidate compound with the expression system for said polypeptide factor and/or the system, preferably an activity system, detecting activity of said polypeptide factor;
d) determining if the expression and/or the activity of said polypeptide factor is changed under the influence of the candidate compound.

24. Method according to claim 23, **characterised in that** the candidate compound is contained in a library of compounds.

25. The method according to claim 23 or 24, **characterised in that** the candidate compound is selected from the group of classes of compounds consisting of high affinity binding peptides, proteins, antibodies, anticalines, functional nucleic acids, natural compounds and small molecules.

26. The method according to claim 25, **characterised in that** the functional nucleic acids are selected from the group which comprises aptameres, aptazymes, ribozymes, spiegelmers, antisense oligonucleotides and siRNA.

27. In vitro use of the polypeptide factor or a fragment as defined in claim 1 as a marker of PI3-kinase activity.

28. In vitro use of a nucleic acid as defined in claim 6 as a marker of PI3-kinase activity.

29. Use of an antisense nucleic acid or an interfering RNA for the manufacture of a medicament for the treatment of a disease, whereby such disease is cancer and the antisense nucleic acid and the interfering RNA are directed against a nucleic acid as defined in claim 6.

30. Use according to claim 29, wherein the cancer is selected from the group consisting of colorectal carcinomas, gliomas, endometrial cancers, adenocarcinomas, endometrial hyperplasias, Cowden's syndrome, breast-ovarian cancer, prostate cancer, Bannayan-Zonana syndrome, LDD (Lhermitte-Duclos' syndrome) hamartoma-macrocephaly diseases including Cowden's disease (CD) and Bannayan-Ruvalcaba-Riley syndrome (BRR), trichilemmomas, gastrointestinal hamartomas, lipomas, thyroid adenomas, fibrocystic disease of the breast, cerebellar dysplastic gangliocytoma and breast and thyroid malignancies

## Patentansprüche

1. In-vitro-Verwendung eines Polypeptidfaktors oder eines Fragmentes davon, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist, wobei der Faktor
a) eine Aminosäuresequenz gemäß SEQ ID NO. 1 umfasst;
b) eine Aminosäuresequenz gemäß den Datenbankeinträgen gi 9506687 oder NP_061931.1 aufweist;
c) durch eine Nukleinsäure codiert ist, wobei die
ca) Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
cb) die Nukleinsäure für den Faktor gemäß a) oder b) codiert und wobei die Nukleinsäure ohne die Degeneriertheit des genetischen Codes an eine Nukleinsäure gemäß ca) hybridisieren würde; oder
cc) die Nukleinsäure für den Faktor gemäß a) oder b) codiert und wobei die Nukleinsäure unter stringenten Bedingungen an eine Nukleinsäure hybridisiert, die komplementär zu der Nukleinsäure gemäß ca) ist;
als ein Downstream-Zielmolekül oder einen Downstream-Marker des PI 3-Kinase-Weges, bevorzugterweise als ein Downstream-Wirkstoff-Zielmolekül des PI 3-Kinase-Weges, beim Screenen auf Wirkstoffe, dem Designen von Wirkstoffen und der Entwicklung von Wirkstoffen, wobei der Wirkstoff nützlich ist bei Wundheilung, Inhibieren von Tumorwachstum, Tumorgenese, Metastase, Zellmigration, Zellmotilität in extrazellulärer Matrix und Zellwachstum in extrazellulärer Matrix.

2. In-vitro-Verwendung eines Polypeptidfaktors, wobei der Faktor
a) eine Aminosäuresequenz gemäß SEQ ID NO. 1 umfasst;
b) eine Aminosäuresequenz gemäß den Datenbankeinträgen gi 9506687 oder NP_061931.1 aufweist;
c) durch eine Nukleinsäure codiert ist, wobei die
ca) Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
cb) die Nukleinsäure für den Faktor gemäß a) oder b) codiert und wobei die Nukleinsäure ohne die Degeneriertheit des genetischen Codes an eine Nukleinsäure gemäß ca) hybridisieren würde; oder
cc) die Nukleinsäure für den Faktor gemäß a) oder b) codiert und wobei die Nukleinsäure unter stringenten Bedingungen an eine Nukleinsäure hybridisiert, die komplementär zu der Nukleinsäure gemäß ca) ist;
als ein Marker für einen Prozess, wobei der Prozess ein PI 3-Kinase-Weg-regulierter Prozess ist, wobei der Prozess ausgewählt ist aus der Gruppe bestehend aus Wundheilung, Metastase, Zellmigration, Zellmotilität in extrazellulärer Matrix und Zellwachstum in extrazellulärer Matrix.

3. Verwendung nach Anspruch 2, wobei der Faktor ein Marker für transformierte Zellen, bevorzugterweise invasive Zellen ist.

4. Verwendung eines Polypeptidfaktors oder eines Fragmentes davon, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist, wobei der Faktor
a) eine Aminosäuresequenz gemäß SEQ ID NO. 1 umfasst;
b) eine Aminosäuresequenz gemäß den Datenbankeinträgen gi 9506687 oder NP_061931.1 aufweist;
c) durch eine Nukleinsäure codiert ist, wobei die
ca) Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM 019058.1 umfasst;
cb) die Nukleinsäure für den Faktor gemäß a) oder b) codiert und wobei die Nukleinsäure ohne die Degeneriertheit des genetischen Codes an eine Nukleinsäure gemäß ca) hybridisieren würde; oder
cc) die Nukleinsäure für den Faktor gemäß a) oder b) codiert und wobei die Nukleinsäure unter stringenten Bedingungen an eine Nukleinsäure hybridisiert, die komplementär zu der Nukleinsäure gemäß ca) ist;
für die Herstellung eines Medikaments für die Behandlung und/oder Prävention einer Erkrankung,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament für die Wundheilung ist.

5. Verwendung eines Polypeptidfaktors oder eines Fragments davon, wobei das Fragment die Wirkungen des Polypeptids hinsichtlich Wundheilung, Tumorwachstum, Tumorgenese, Metastase, Zellmigration, Zellmotilität in extrazellulärer Matrix und Zellwachstum in extrazellulärer Matrix aufweist, wobei der Faktor
a) eine Aminosäuresequenz gemäß SEQ ID NO. 1 umfasst;
b) eine Aminosäuresequenz gemäß den Datenbankeinträgen gi 9506687 oder NP_061931.1 aufweist;
c) durch eine Nukleinsäure codiert ist, wobei die
ca) Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
cb) die Nukleinsäure für den Faktor gemäß a) oder b) codiert und wobei die Nukleinsäure ohne die Degeneriertheit des genetischen Codes an eine Nukleinsäure gemäß ca) hybridisieren würde; oder
cc) die Nukleinsäure für den Faktor gemäß a) oder b) codiert und wobei die Nukleinsäure unter stringenten Bedingungen an eine Nukleinsäure hybridisiert, die komplementär zu der Nukleinsäure gemäß ca) ist;
für die Herstellung eines diagnostischen Mittels für die Diagnose einer Erkrankung,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament für die Wundheilung ist.

6. Verwendung einer Nukleinsäure, wobei die Nukleinsäure
a) eine Nukleinsäure ist, die für einen Faktor oder ein Fragment davon codiert, wobei der Faktor
i) ein Polypeptid ist umfassend eine Aminosäuresequenz gemäß SEQ ID NO. 1 ; oder
ii) ein Polypeptid mit einer Sequenz gemäß den Datenbankeinträgen gi 9506687 oder NM_061931.1;
b) eine Nukleinsäure ist, die für den Faktor gemäß a) codiert, wobei die Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
c) eine Nukleinsäure ist, die für den Faktor gemäß a) codiert, wobei die Nukleinsäure ohne die Degeneriertheit des genetischen Codes an eine Nukleinsäure gemäß b) hybridisieren würde; oder
d) eine Nukleinsäure ist, die für den Faktor gemäß a) codiert, wobei die Nukleinsäure unter stringenten Bedingungen an eine Nukleinsäure hybridisiert, die komplementär zu der Nukleinsäure gemäß b) ist;
für die Herstellung eines Medikaments für die Behandlung und/oder Prävention einer Erkrankung, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament für die Wundheilung ist.

7. Verwendung einer Nukleinsäure, wobei die Nukleinsäure
a) eine Nukleinsäure ist, die für einen Faktor oder ein Fragment davon codiert, wobei der Faktor
i) ein Polypeptid ist umfassend eine Aminosäuresequenz gemäß SEQ ID NO. 1 ; oder
ii) ein Polypeptid mit einer Sequenz gemäß den Datenbankeinträgen gi 9506687 oder NM_061931.1;
b) eine Nukleinsäure ist, die für den Faktor gemäß a) codiert, wobei die Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
c) eine Nukleinsäure ist, die für den Faktor gemäß a) codiert, wobei die Nukleinsäure ohne die Degeneriertheit des genetischen Codes an eine Nukleinsäure gemäß b) hybridisieren würde; oder
d) eine Nukleinsäure ist, die für den Faktor gemäß a) codiert, wobei die Nukleinsäure unter stringenten Bedingungen an eine Nukleinsäure hybridisiert, die komplementär zu der Nukleinsäure gemäß b) ist;
für die Herstellung eines diagnostischen Agens für die Diagnose einer Erkrankung, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), mukokutanen Läsionen wie Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament für die Wundheilung ist.

8. Verwendung nach einem der Ansprüche 1 und 3 bis 7, wobei
die Erkrankung **dadurch gekennzeichnet ist, dass** den in dieser Erkrankung involvierten Zellen PTEN-Aktivität fehlt oder sie eine Hyperaktivierung des PI 3-Kinase-Weges zeigen, oder ein erhöhtes aggressives Verhalten zeigen, oder Tumorzellen sind, bevorzugterweise Zellen eines Spätphasentumors.

9. In-vitro-Verwendung eines Polypeptidfaktors oder eines Fragments davon, wobei der Faktor
a) eine Aminosäuresequenz gemäß SEQ ID NO. 1 umfasst;
b) eine Aminosäuresequenz gemäß den Datenbankeinträgen gi 9506687 oder NP_061931.1 aufweist;
c) durch eine Nukleinsäure codiert ist, wobei die
ca) Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
cb) die Nukleinsäure für den Faktor gemäß a) oder b) codiert und wobei die Nukleinsäure ohne die Degeneriertheit des genetischen Codes an eine Nukleinsäure gemäß ca) hybridisieren würde; oder
cc) die Nukleinsäure für den Faktor gemäß a) oder b) codiert und wobei die Nukleinsäure unter stringenten Bedingungen an eine Nukleinsäure hybridisiert, die komplementär zu der Nukleinsäure gemäß ca) ist;
als Zielmolekül für die Entwicklung eines Medikaments für die Behandlung und/oder Prävention einer Erkrankung, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament für die Wundheilung ist.

10. In-vitro-Verwendung eines Polypeptidfaktors oder eines Fragments davon, wobei der Faktor
a) eine Aminosäuresequenz gemäß SEQ ID NO. 1 umfasst;
b) eine Aminosäuresequenz gemäß den Datenbankeinträgen gi 9506687 oder NP_061931.1 aufweist;
c) durch eine Nukleinsäure codiert ist, wobei die
ca) Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
cb) die Nukleinsäure für den Faktor gemäß a) oder b) codiert und wobei die Nukleinsäure ohne die Degeneriertheit des genetischen Codes an eine Nukleinsäure gemäß ca) hybridisieren würde; oder
cc) die Nukleinsäure für den Faktor gemäß a) oder b) codiert und wobei die Nukleinsäure unter stringenten Bedingungen an eine Nukleinsäure hybridisiert, die komplementär zu der Nukleinsäure gemäß ca) ist;
als ein Zielmolekül für die Entwicklung eines diagnostischen Agens für die Diagnose einer Erkrankung, wobei das Fragment oder Derivat die Wirkungen des Poylpeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das diagnostische Agens für die Wundheilung ist.

11. In-vitro-Verwendung einer Nukleinsäure, wobei die Nukleinsäure
a) eine Nukleinsäure ist, die für einen Faktor oder ein Fragment davon codiert, wobei der Faktor
i) ein Polypeptid ist umfassend eine Aminosäuresequenz gemäß SEQ ID NO. 1 ; oder
ii) ein Polypeptid mit einer Sequenz gemäß den Datenbankeinträgen gi 9506687 oder NM_061931.1;
b) eine Nukleinsäure ist, die für den Faktor gemäß a) codiert, wobei die Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
c) eine Nukleinsäure ist, die für den Faktor gemäß a) codiert, wobei die Nukleinsäure ohne die Degeneriertheit des genetischen Codes an eine Nukleinsäure gemäß b) hybridisieren würde; oder
d) eine Nukleinsäure ist, die für den Faktor gemäß a) codiert, wobei die Nukleinsäure unter stringenten Bedingungen an eine Nukleinsäure hybridisiert, die komplementär zu der Nukleinsäure gemäß b) ist;
als ein Zielmolekül für die Entwicklung eines Medikaments für die Behandlung und/oder Prävention einer Erkrankung, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament für die Wundheilung ist.

12. In-vitro-Verwendung einer Nukleinsäure, wobei die Nukleinsäure
a) eine Nukleinsäure ist, die für einen Faktor oder ein Fragment davon codiert, wobei der Faktor
i) ein Polypeptid ist umfassend eine Aminosäuresequenz gemäß SEQ ID NO. 1 ; oder
ii) ein Polypeptid mit einer Sequenz gemäß den Datenbankeinträgen gi 9506687 oder NM_061931.1;
b) eine Nukleinsäure ist, die für den Faktor gemäß a) codiert, wobei die Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
c) eine Nukleinsäure ist, die für den Faktor gemäß a) codiert, wobei die Nukleinsäure ohne die Degeneriertheit des genetischen Codes an eine Nukleinsäure gemäß b) hybridisieren würde; oder
d) eine Nukleinsäure ist, die für den Faktor gemäß a) codiert, wobei die Nukleinsäure unter stringenten Bedingungen an eine Nukleinsäure hybridisiert, die komplementär zu der Nukleinsäure gemäß b) ist;
als ein Zielmolekül für die Entwicklung eines diagnostischen Agens für die Diagnose einer Erkrankung, wobei das Fragment oder Derivat die Wirkungen des Poylpeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das diagnostische Agens für die Wundheilung ist.

13. Verwendung gemäß einem der Ansprüche 9 bis 12, **dadurch gekennzeichnet, dass** das Medikament und/oder das diagnostische Agens ein Agens umfasst, das ausgewählt ist aus der Gruppe bestehend aus Antikörpern, hochaffin bindenden Peptiden, Antikalinen, Antisense-Molekülen, Aptameren, Spiegelmeren und RNAi-Molekülen.

14. Verwendung nach Anspruch 13, **dadurch gekennzeichnet, dass** das Agens mit einem Faktor oder einem Fragment davon wie in Anspruch 1 definiert wechselwirkt.

15. Verwendung nach Anspruch 13, **dadurch gekennzeichnet, dass** das Agens mit der Nukleinsäure in Wechselwirkung tritt, wie in Anspruch 6 definiert, insbesondere mit mRNA, genomischer Nukleinsäure oder cDNA, die für den Polypeptidfaktor wie in Anspruch 1 definiert codiert.

16. Verwendung eines Antikörpers oder Teils davon, der mit dem Polypeptidfaktor oder dem Fragment davon wie in Anspruch 1 definiert, wechselwirkt, für die Entwicklung oder Herstellung eines Medikamentes für die Behandlung und/oder Prävention einer Erkrankung und/oder für die Herstellung eines diagnostischen Agens für die Diagnose einer Erkrankung,
wobei die Erkrankung ausgewählt ist aus der Gruppe bestehend aus Spätphasentumor, metastatischem Krebs und jeglichen pathologischen Zuständen, die den PI 3-Kinase-Weg involvieren, wobei derartige Zustände ausgewählt sind aus der Gruppe bestehend aus
Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament und/oder das diagnostische Agens für die Wundheilung ist/sind.

17. Verwendung eines Aptamers oder Spiegelmers, welches mit dem Polypeptidfaktor oder Fragment davon, wie in Anspruch 1 definiert, wechselwirkt, für die Entwicklung oder Herstellung eines diagnostischen Agens für die Diagnose einer Erkrankung,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das diagnostische Agens für die Wundheilung ist.

18. Verwendung einer Nukleinsäure, die mit einer Nukleinsäure, die für den Polypeptidfaktor oder ein Fragment davon codiert, wie in Anspruch 1 definiert, wechselwirkt, für die Entwicklung oder Herstellung eines Medikaments für die Behandlung und/oder Prävention einer Erkrankung und/oder für die Herstellung eines diagnostischen Agens für die Diagnose einer Erkrankung,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament und/oder das diagnostische Agens für die Wundheilung ist/sind,
wobei die wechselwirkende Nukleinsäure ein Antisense-Oligonukleotid, ein Ribozym und/oder siRNA ist.

19. Verwendung nach Anspruch 18, **dadurch gekennzeichnet, dass** die Nukleinsäure, die für den Polypeptidfaktor oder einen Teil davon codiert, cDNA, mRNA oder hnRNA ist.

20. Verwendung eines Kits für die Charakterisierung einer Erkrankung oder eines Zustandes, die/der ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
wobei das Kit wenigstens ein Agens umfasst, welches ausgewählt ist aus der Gruppe bestehend aus
a) dem Polypeptidfaktor oder Fragment davon, wie in Anspruch 1 definiert,
b) Antikörpern oder Teilen davon, die für den Polypeptidfaktor oder das Fragment davon gemäß a) spezifisch sind,
c) einer Nukleinsäure, die für einen Faktor codiert, wobei der Faktor
i) ein Polypeptid ist umfassend eine Aminosäure gemäß SEQ ID NO. 1 ; oder
ii) ein Polypeptid mit einer Sequenz gemäß den Datenbankeinträgen gi 9506687 oder NM 061931.1;
d) einer Nukleinsäure, die für den Faktor oder das Fragment davon gemäß a) codiert, wobei die Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058.1 umfasst;
e) einer Nukleinsäure, die für den Faktor oder das Fragment gemäß a) codiert, wobei die Nukleinsäure ohne die Degeneriertheit des genetischen Codes an eine Nukleinsäure gemäß d) hybridisieren würde; oder
f) einer Nukleinsäure, die für den Faktor oder das Fragment davon gemäß a) codiert, wobei die Nukleinsäure unter stringenten Bedingungen an eine Nukleinsäure hybridisiert, die komplementär zu der Nukleinsäure gemäß d) ist;
g) Nukleinsäuren, die mit dem Polypeptidfaktor oder Fragment davon wechselwirkt, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist, und wobei die Nukleinsäuren ausgewählt sind aus der Gruppe bestehend aus Aptameren und Spiegelmeren, und
h) Nukleinsäuren, die wechselwirken mit
ha) einer Nukleinsäure codierend für einen Faktor, wobei der Faktor
i) ein Polypeptid ist umfassend eine Aminosäure gemäß SEQ ID NO. 1; oder
ii) ein Polypeptid mit einer Aminosäuresequenz gemäß den Datenbankeinträgen gi 9506687 oder NP_061931 ist;
hb) einer Nukleinsäure, die für einen Faktor codiert, wobei die Nukleinsäure
i) eine Nukleinsäure gemäß SEQ ID NO. 2 oder SEQ ID NO. 3; oder
ii) eine Nukleinsäuresequenz gemäß den Datenbankeinträgen gi 9506686 oder NM_019058 umfasst;
hc) einer Nukleinsäure, die für den Faktor oder ein Fragment davon gemäß a) codiert, wobei die Nukleinsäure ohne die Degeneriertheit des genetischen Codes an eine Nukleinsäure gemäß d) hybridisieren würde; oder
hd) eine Nukleinsäure, die für den Faktor oder ein Fragment davon gemäß a) codiert, wobei die Nukleinsäure unter stringenten Bedingungen an eine Nukleinsäure hybridisiert, die komplementär zu der Nukleinsäure gemäß d) ist;
und optional wenigstens eine weitere Verbindung.

21. Verwendung nach einem der Ansprüche 1 bis 20, wobei der Polypeptidfaktor an einem biologischen Prozess beteiligt ist, wobei der Prozess ein PI 3-Kinase-Weg-regulierter Prozess ist.

22. Verwendung nach Anspruch 21, wobei der Prozess
ein Prozess ist, der ausgewählt ist aus der Gruppe bestehend aus Wundheilung, Metastase, Tumorgenese, Zellmigration, Zellmotilität in extrazellulärer Matrix und Zellwachstum in extrazellulärer Matrix.

23. Verfahren zum Screenen eines Agens für die Herstellung eines Medikaments für die Behandlung und/oder Prävention einer Erkrankung und/oder für das Screenen eines Agens für die Herstellung eines diagnostischen Agens für die Diagnose einer Erkrankung,
wobei die Erkrankung ausgewählt ist aus der Gruppe bestehend aus Spätphasentumor, metastatischem Krebs und jeglichen pathologischen Zuständen, die den PI 3-Kinase-Weg involvieren,
wobei derartige Zustände ausgewählt sind aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten,
oder wobei das Medikament und/oder das diagnostische Agens für die Wundheilung ist/sind,
umfassend die folgenden Schritte:
a) Bereitstellen einer Kandidatenverbindung,
b) Bereitstellen eines Expressionssystems für einen Polypeptidfaktor oder ein Fragment davon, wobei das Fragment die Wirkungen des Polypeptidfaktors hinsichtlich Wundheilung, Tumorwachstum, Tumorgenese, Metastase, Zellmigration, Zellmotilität in extrazellulärer Matrix und Zellwachstum in extrazellulärer Matrix aufweist, wobei der Faktor
ba) eine Aminosäuresequenz gemäß SEQ ID NO. 1 umfasst;
bb) eine Aminosäuresequenz gemäß Datenbankeinträgen gi 9506687 oder NP_061931.1 aufweist;
bc) durch eine Nukleinsäure codiert ist, wobei die
bca) Nukleinsäure
i) eine Nukleinsäure gemäß SEQ ID NO. 2 oder SEQ ID NO. 3; oder
ii) eine Nukleinsäuresequenz gemäß Datenbankeinträgen gi 9506686 oder NM_019058.1 umfasst;
bcb) die Nukleinsäure für den Faktor oder das Fragment davon gemäß b), ba) oder bb) codiert und wobei die Nukleinsäure ohne die Degeneriertheit des genetischen Codes an eine Nukleinsäure gemäß bca) hybridisieren würde; oder
bcc) eine Nukleinsäure für den Faktor oder das Fragment davon gemäß b), ba) oder bb) codiert und wobei die Nukleinsäure unter stringenten Bedingungen an eine Nukleinsäure hybridisiert, die komplementär zu der Nukleinsäure gemäß bca) ist;
und/oder ein System, bevorzugterweise ein Aktivitätssystem, das die Aktivität des Polypeptidfaktors nachweist;
c) Kontaktieren der Kandidatenverbindung mit dem Expressionssystem für den Polypeptidfaktor und/oder dem System, bevorzugterweise einem Aktivitätssystem, welches die Aktivität des Polypeptidfaktors nachweist;
d) Bestimmen, ob die Expression und/oder Aktivität des Polypeptidfaktors unter dem Einfluss der Kandidatenverbindung geändert ist.

24. Verfahren nach Anspruch 23, **dadurch gekennzeichnet, dass** die Kandidatenverbindung in einer Verbindungsbibliothek enthalten ist.

25. Verfahren nach Anspruch 23 oder 24, **dadurch gekennzeichnet, dass** die Kandidatenverbindung ausgewählt ist aus der Gruppe von Verbindungsklassen bestehend aus hochaffin bindenden Peptiden, Proteinen, Antikörpern, Antikalinen, funktionalen Nukleinsäuren, natürlichen Verbindungen und kleinen Molekülen.

26. Verfahren nach Anspruch 25, **dadurch gekennzeichnet, dass** die funktionalen Nukleinsäuren ausgewählt sind aus der Gruppe umfassend Aptamere, Aptazyme, Ribozyme, Spiegelmere, Antisense-Oligonukleotide und siRNA.

27. In-vitro-Verwendung des Polypeptidfaktors oder eines Fragmentes wie in Anspruch 1 definiert, als Marker für PI 3-Kinase-Aktivität.

28. In-vitro-Verwendung einer Nukleinsäure wie in Anspruch 6 definiert, als Marker für PI3-Kinase-Aktivität.

29. Verwendung einer Antisense-Nukleinsäure oder einer interferierenden RNA für die Herstellung eines Medikaments für die Behandlung einer Erkrankung, wobei eine derartige Erkrankung Krebs ist und die Antisense-Nukleinsäure und die interferierende RNA gegen eine Nukleinsäure gerichtet sind, wie sie in Anspruch 6 definiert ist.

30. Verwendung nach Anspruch 29, wobei der Krebs ausgewählt ist aus der Gruppe bestehend aus Kolorektalkarzinomen, Gliomen, Endometriumkarzinomen, Adenokarzinomen, Endometriumhyperplasien, Cowden-Syndrom, Brust-Ovarial-Karzinom, Prostatakrebs, Bannayan-Riley-Ruvalcaba-Syndrom, LDD (Lhermitte-Duclos-Syndrom), Hamartom-Makrozephalie-Erkrankungen einschließlich Cowden-Syndrom (CD) und Bannayan-Riley-Ruvalcaba-Syndrom (BRR), Trichilemmomen, gastrointestinalen Hamartomen, Lipomen, Schilddrüsenadenomen, fibrozystischer Erkrankung der Brust, zerebellärem dysplastischem Gangliozytom und Brust- und Schilddrüsenmalignitäten.

## Revendications

1. Utilisation in vitro d'un facteur de type polypeptide ou d'un fragment de celui-ci, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire, moyennant quoi le facteur
a) comprend une séquence d'acides aminés définie selon SEQ ID NO. 1 ;
b) a une séquence d'acides aminés définie selon les entrées de la banque de données gi 9506687 ou NP 061931.1 ;
c) est codé par un acide nucléique, moyennant quoi
ca) l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
cb) l'acide nucléique encode pour le facteur comme pour a) ou b) et moyennant quoi l'acide nucléique devrait s'hybrider mais pour la dégénérescence du code génétique, en un acide nucléique défini selon ca) ; ou
cc) l'acide nucléique encode pour le facteur comme pour a) ou b) et moyennant quoi l'acide nucléique s'hybride dans des conditions stringentes en un acide nucléique qui est complémentaire de l'acide nucléique défini selon ca) ;
en tant qu'une cible en aval ou un marqueur en aval du chemin de la PI 3-kinase, de préférence en tant qu'une cible de médicament en aval du chemin de la PI 3-kinase, dans le criblage d'un médicament, la conception d'un médicament et le développement d'un médicament, moyennant quoi le médicament est utile pour la cicatrisation de plaie, l'inhibition de la croissance des tumeurs, l'oncogenèse, la métastase, la migration de cellules, la motilité de cellules dans une matrice extracellulaire et la croissance de cellules dans une matrice extracellulaire.

2. Utilisation in vitro d'un facteur de type polypeptide, moyennant quoi le facteur
a) comprend une séquence d'acides aminés définie selon SEQ ID NO. 1 ;
b) a une séquence d'acides aminés définie selon les entrées de la banque de données gi 9506687 ou NP 061931.1 ;
c) est codé par un acide nucléique, moyennant quoi
ca) l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
cb) l'acide nucléique encode pour le facteur comme pour a) ou b) et moyennant quoi l'acide nucléique devrait s'hybrider mais pour la dégénérescence du code génétique, en un acide nucléique défini selon ca) ; ou
cc) l'acide nucléique encode pour le facteur comme pour a) ou b) et moyennant quoi l'acide nucléique s'hybride dans des conditions stringentes en un acide nucléique qui est complémentaire de l'acide nucléique défini selon ca) ;
en tant qu'un marqueur pour un processus, moyennant quoi le processus est un processus régulé du chemin de la PI 3-kinase, moyennant quoi le processus est choisi dans le groupe constitué par la cicatrisation de plaie, la métastase, la migration de cellules, la motilité de cellules dans une matrice extracellulaire et la croissance de cellules dans une matrice extracellulaire.

3. Utilisation selon la revendication 2, moyennant quoi le facteur est un marqueur pour des cellules transformées, de préférence pour des cellules envahissantes.

4. Utilisation d'un facteur de type polypeptide ou d'un fragment de celui-ci, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire, moyennant quoi le facteur
a) comprend une séquence d'acides aminés définie selon SEQ ID NO. 1 ;
b) a une séquence d'acides aminés définie selon les entrées de la banque de données gi 9506687 ou NP 061931.1 ;
c) est codé par un acide nucléique, moyennant quoi
ca) l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
cb) l'acide nucléique encode pour le facteur comme pour a) ou b) et moyennant quoi l'acide nucléique devrait s'hybrider mais pour la dégénérescence du code génétique, en un acide nucléique défini selon ca) ; ou
cc) l'acide nucléique encode pour le facteur comme pour a) ou b) et moyennant quoi l'acide nucléique s'hybride dans des conditions stringentes en un acide nucléique qui est complémentaire de l'acide nucléique défini selon ca) ;
pour la fabrication d'un médicament destiné au traitement et/ou à la prévention d'une maladie,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le médicament est destiné à la cicatrisation de plaie.

5. Utilisation d'un facteur de type polypeptide ou d'un fragment de celui-ci, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire, moyennant quoi le facteur
a) comprend une séquence d'acides aminés définie selon SEQ ID NO. 1 ;
b) a une séquence d'acides aminés définie selon les entrées de la banque de données gi 9506687 ou NP 061931.1 ;
c) est codé par un acide nucléique, moyennant quoi
ca) l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
cb) l'acide nucléique encode pour le facteur comme pour a) ou b) et moyennant quoi l'acide nucléique devrait s'hybrider mais pour la dégénérescence du code génétique, en un acide nucléique défini selon ca) ; ou
cc) l'acide nucléique encode pour le facteur comme pour a) ou b) et moyennant quoi l'acide nucléique s'hybride dans des conditions stringentes en un acide nucléique qui est complémentaire de l'acide nucléique défini selon ca) ;
pour la fabrication d'un agent diagnostique destiné au diagnostic d'une maladie,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi l'agent diagnostique est destiné à la cicatrisation de plaie.

6. Utilisation d'un acide nucléique, moyennant quoi un tel acide nucléique est
a) un acide nucléique codant pour un facteur ou un fragment de celui-ci, moyennant quoi le facteur est
i) un polypeptide comprenant un acide aminé défini selon SEQ ID NO. 1 ; ou
ii) un polypeptide ayant une séquence définie selon les entrées de la banque de données gi 9506687 ou NP 061931.1 ;
b) un acide nucléique codant pour le facteur comme pour a), moyennant quoi l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
c) un acide nucléique codant pour le facteur comme pour a), moyennant quoi l'acide nucléique devrait s'hybrider mais pour la dégénérescence du code génétique, en un acide nucléique défini selon b) ;
ou
d) un acide nucléique codant pour le facteur comme pour a), moyennant quoi l'acide nucléique s'hybride dans des conditions stringentes en un acide nucléique qui est complémentaire de l'acide nucléique défini selon b) ;
pour la fabrication d'un médicament destiné au traitement et/ou à la prévention d'une maladie, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le médicament est destiné à la cicatrisation de plaie.

7. Utilisation d'un acide nucléique, moyennant quoi un tel acide nucléique est
a) un acide nucléique codant pour un facteur ou un fragment de celui-ci, moyennant quoi le facteur est
i) un polypeptide comprenant un acide aminé défini selon SEQ ID NO. 1 ; ou
ii) un polypeptide ayant une séquence définie selon les entrées de la banque de données gi 9506687 ou NP 061931.1 ;
b) un acide nucléique codant pour le facteur comme pour a), moyennant quoi l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
c) un acide nucléique codant pour le facteur comme pour a), moyennant quoi l'acide nucléique devrait s'hybrider mais pour la dégénérescence du code génétique, en un acide nucléique défini selon b) ; ou
d) un acide nucléique codant pour le facteur comme pour a), moyennant quoi l'acide nucléique s'hybride dans des conditions stringentes en un acide nucléique qui est complémentaire de l'acide nucléique défini selon b) ;
pour la fabrication d'un agent diagnostique destiné au diagnostic d'une maladie, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), des lésions mucocutanées, telles que les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi l'agent diagnostique est destiné à la cicatrisation de plaie.

8. Utilisation selon l'une quelconque des revendications 1 et 3 à 7, moyennant quoi
la maladie est **caractérisée en ce que** les cellules impliquées dans ladite maladie manquent d'activité PTEN, ou présentent une hyperactivation du chemin de la PI 3-kinase, ou présentent un comportement agressif accru ou sont des cellules tumorales, de préférence des cellules à un stade avancé.

9. Utilisation in vitro d'un facteur de type polypeptide ou d'un fragment de celui-ci, moyennant quoi le facteur
a) comprend une séquence d'acides aminés définie selon SEQ ID NO. 1 ;
b) a une séquence d'acides aminés définie selon les entrées de la banque de données gi 9506687 ou NP 061931.1 ;
c) est codé par un acide nucléique, moyennant quoi
ca) l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
cb) l'acide nucléique encode pour le facteur comme pour a) ou b) et moyennant quoi l'acide nucléique devrait s'hybrider mais pour la dégénérescence du code génétique, en un acide nucléique défini selon ca) ; ou
cc) l'acide nucléique encode pour le facteur comme pour a) ou b) et moyennant quoi l'acide nucléique s'hybride dans des conditions stringentes en un acide nucléique qui est complémentaire de l'acide nucléique défini selon ca) ;
en tant qu'une molécule cible pour le développement d'un médicament destiné au traitement et/ou à la prévention d'une maladie, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le médicament est destiné à la cicatrisation de plaie.

10. Utilisation in vitro d'un facteur de type polypeptide ou d'un fragment de celui-ci, moyennant quoi le facteur
a) comprend une séquence d'acides aminés définie selon SEQ ID NO. 1 ;
b) a une séquence d'acides aminés définie selon les entrées de la banque de données gi 9506687 ou NP 061931.1 ;
c) est codé par un acide nucléique, moyennant quoi
ca) l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
cb) l'acide nucléique encode pour le facteur comme pour a) ou b) et moyennant quoi l'acide nucléique devrait s'hybrider mais pour la dégénérescence du code génétique, en un acide nucléique défini selon ca) ; ou
cc) l'acide nucléique encode pour le facteur comme pour a) ou b) et moyennant quoi l'acide nucléique s'hybride dans des conditions stringentes en un acide nucléique qui est complémentaire de l'acide nucléique défini selon ca) ;
en tant qu'une molécule cible pour le développement d'un agent diagnostique destiné au diagnostic d'une maladie, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi l'agent diagnostique est destiné à la cicatrisation de plaie.

11. Utilisation in vitro d'un acide nucléique, moyennant quoi un tel acide nucléique est
a) un acide nucléique codant pour un facteur ou un fragment de celui-ci, moyennant quoi le facteur est
i) un polypeptide comprenant un acide aminé défini selon SEQ ID NO. 1 ; ou
ii) un polypeptide ayant une séquence définie selon les entrées de la banque de données gi 9506687 ou NP 061931.1 ;
b) un acide nucléique codant pour le facteur comme pour a), moyennant quoi l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
c) un acide nucléique codant pour le facteur comme pour a), moyennant quoi l'acide nucléique devrait s'hybrider mais pour la dégénérescence du code génétique, en un acide nucléique défini selon b) ; ou
d) un acide nucléique codant pour le facteur comme pour a), moyennant quoi l'acide nucléique s'hybride dans des conditions stringentes en un acide nucléique qui est complémentaire de l'acide nucléique défini selon b) ;
en tant qu'une molécule cible pour le développement d'un médicament destiné au traitement et/ou à la prévention d'une maladie, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le médicament est destiné à la cicatrisation de plaie.

12. Utilisation in vitro d'un acide nucléique, moyennant quoi un tel acide nucléique est
a) un acide nucléique codant pour un facteur ou un fragment de celui-ci, moyennant quoi le facteur est
i) un polypeptide comprenant un acide aminé défini selon SEQ ID NO. 1 ; ou
ii) un polypeptide ayant une séquence définie selon les entrées de la banque de données gi 9506687 ou NP 061931.1 ;
b) un acide nucléique codant pour le facteur comme pour a), moyennant quoi l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
c) un acide nucléique codant pour le facteur comme pour a), moyennant quoi l'acide nucléique devrait s'hybrider mais pour la dégénérescence du code génétique, en un acide nucléique défini selon b) ; ou
d) un acide nucléique codant pour le facteur comme pour a), moyennant quoi l'acide nucléique s'hybride dans des conditions stringentes en un acide nucléique qui est complémentaire de l'acide nucléique défini selon b) ;
en tant qu'une molécule cible pour le développement d'un agent diagnostique destiné au diagnostique d'une maladie, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi l'agent diagnostique est destiné à la cicatrisation de plaie.

13. Utilisation selon l'une quelconque des revendications 9 à 12, **caractérisée en ce que** le médicament et/ou l'agent diagnostique comprend un agent qui est choisi dans le groupe constitué par des anticorps, des peptides à liaison de grande affinité, des anticalins, des molécules antisens, des aptamères, des spiegelmères et des molécules d'ARNi.

14. Utilisation selon la revendication 13, **caractérisée en ce que** l'agent interagit avec le facteur ou un fragment de celui-ci défini selon la revendication 1.

15. Utilisation selon la revendication 13, **caractérisée en ce que** l'agent interagit avec l'acide nucléique défini selon la revendication 6, en particulier avec le mARN, un acide nucléique génomique ou le cADN codant pour le facteur de type polypeptide défini selon la revendication 1.

16. Utilisation d'un anticorps ou d'une partie de celui-ci qui interagit avec le facteur de type polypeptide ou le fragment de celui-ci tel que défini selon la revendication 1, pour le développement ou la fabrication d'un médicament destiné au traitement et/ou à la prévention d'une maladie et/ou pour la fabrication d'un agent diagnostique destiné au diagnostic d'une maladie,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le médicament et/ou l'agent diagnostique sont destinés à la cicatrisation de plaie.

17. Utilisation d'un aptamère ou spiegelmère qui interagit avec le facteur de type polypeptide ou un fragment de celui-ci tel que défini selon la revendication 1, pour le développement ou la fabrication d'un agent diagnostique destiné au diagnostique d'une maladie,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi l'agent diagnostique est destiné à la cicatrisation de plaie.

18. Utilisation d'un acide nucléique qui interagit avec un acide nucléique codant pour le facteur de type polypeptide ou un fragment de celui-ci tel que défini selon la revendication 1, pour le développement ou la fabrication d'un médicament destiné au traitement et/ou à la prévention d'une maladie et/ou pour la fabrication d'un agent diagnostique destiné au diagnostique d'une maladie,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le médicament et/ou l'agent diagnostique sont destinés à la cicatrisation de plaie,
moyennant quoi l'acide nucléique interagissant est un oligonucléotide, un ribozyme et/ou le siARN

19. Utilisation selon la revendication 18, **caractérisée en ce que** l'acide nucléique codant pour le facteur de type polypeptide ou une partie de celui-ci est le cADN, le mARN et le hnARN.

20. Utilisation d'un kit destiné à la caractérisation d'une maladie ou d'une condition qui est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le kit comprend au moins un agent qui est choisi dans le groupe constitué par
a) le facteur de type polypeptide ou un fragment de celui-ci tel que défini selon la revendication 1,
b) des anticorps ou une partie de ceux-ci spécifiques pour le facteur de type polypeptide ou le fragment de celui-ci comme pour a),
c) un acide nucléique codant pour un facteur, moyennant quoi le facteur est
i) un polypeptide comprenant un acide aminé défini selon SEQ ID NO. 1 ; ou
ii) un polypeptide ayant une séquence définie selon les entrées de la banque de données gi 9506687 ou NP 061931.1 ;
d) un acide nucléique codant pour le facteur ou un fragment de celui-ci comme pour a), moyennant quoi l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
e) un acide nucléique codant pour le facteur ou le fragment comme pour a), moyennant quoi l'acide nucléique devrait s'hybrider mais pour la dégénérescence du code génétique, en un acide nucléique défini selon d) ;
ou
f) un acide nucléique codant pour le facteur comme pour a), moyennant quoi l'acide nucléique s'hybride dans des conditions stringentes en un acide nucléique qui est complémentaire de l'acide nucléique défini selon d) ;
g) des acides nucléiques interagissant avec ledit polypeptide ou un fragment de celui-ci moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire, et moyennant quoi les acides nucléiques sont choisis dans le groupe constitué par des aptamères et des spiegelmères, et
h) des acides nucléiques interagissant avec
ha) un acide nucléique codant pour un facteur, moyennant quoi le facteur est
i) un polypeptide comprenant un acide aminé défini selon SEQ ID NO. 1 ; ou
ii) un polypeptide ayant une séquence définie selon les entrées de la banque de données gi 9506687 ou NP 061931 ;
hb) un acide nucléique codant pour le facteur, moyennant quoi l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058 ;
hc) un acide nucléique codant pour le facteur ou le fragment comme pour a), moyennant quoi l'acide nucléique devrait s'hybrider mais pour la dégénérescence du code génétique, en un acide nucléique défini selon d) ; ou
hd) un acide nucléique codant pour le facteur comme pour a), moyennant quoi l'acide nucléique s'hybride dans des conditions stringentes en un acide nucléique qui est complémentaire de l'acide nucléique défini selon d) ;
et éventuellement au moins un autre composé.

21. Utilisation selon l'une quelconque des revendications 1 à 20, moyennant quoi le facteur de type polypeptide est impliqué dans un processus biologique, moyennant quoi le processus est un processus régulé du chemin de la PI 3-kinase.

22. Utilisation selon la revendication 21, moyennant quoi le processus est choisi dans le groupe constitué par la cicatrisation de plaie, la métastase, la migration de cellules, la motilité de cellules dans une matrice extracellulaire et la croissance de cellules dans une matrice extracellulaire.

23. Procédé pour le criblage d'un agent pour la fabrication d'un médicament destiné au traitement et/ou à la prévention d'une maladie et/ou pour le criblage d'un agent pour la fabrication d'un agent diagnostique destiné au diagnostic d'une maladie,
moyennant quoi la maladie est choisie dans le groupe constitué par une tumeur à un stade avancé, un cancer métastatique et toutes conditions pathologiques impliquant le chemin de la PI 3-kinase,
moyennant quoi de telles conditions sont choisies dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde,
ou moyennant quoi le médicament et/ou l'agent diagnostique sont destinés à la cicatrisation de plaie,
comprenant les étapes suivantes consistant à :
a) fournir un composé candidat,
b) fournir un système d'expression pour le facteur de type polypeptide ou un fragment de celui-ci, moyennant quoi le fragment a les effets du facteur de type polypeptide en termes de cicatrisation de plaie, de croissance des tumeurs, d'oncogenèse, de métastase, de migration de cellules, de motilité de cellules dans une matrice extracellulaire et de croissance de cellules dans une matrice extracellulaire, moyennant quoi le facteur :
(ba) comprend une séquence d'acides aminés définie selon SEQ ID NO. 1 ;
bb) a une séquence d'acides aminés définie selon les entrées de la banque de données gi 9506687 ou NP 061931.1 ;
bc) est codé par un acide nucléique, moyennant quoi
bca) l'acide nucléique comprend
i) un acide nucléique défini selon SEQ ID NO. 2 ou SEQ ID NO. 3 ; ou
ii) une séquence d'acides nucléiques définie selon les entrées de la banque de données gi 9506686 ou NM 019058.1 ;
bcb) l'acide nucléique encode pour le facteur ou un fragment de celui-ci comme pour b), ba) ou bb) et moyennant quoi l'acide nucléique devrait s'hybrider mais pour la dégénérescence du code génétique, en un acide nucléique défini selon bca) ; ou
bcc) l'acide nucléique encode pour le facteur ou un fragment de celui-ci comme pour b), ba) ou bb) et moyennant quoi l'acide nucléique s'hybride dans des conditions stringentes en un acide nucléique qui est complémentaire de l'acide nucléique défini selon bca) ;
et/ou un système, de préférence un système d'activité, détectant l'activité dudit facteur de type polypeptide ;
c) mettre en contact le composé candidat avec le système d'expression pour ledit facteur de type polypeptide et/ou le système, de préférence un système d'activité, détectant l'activité dudit facteur de type polypeptide ;
d) déterminer si l'expression et/ou l'activité dudit facteur de type polypeptide sont modifiées sous l'influence du composé candidat.

24. Procédé selon la revendication 23, **caractérisé en ce que** le composé candidat est contenu dans une bibliothèque de composés.

25. Procédé selon la revendication 23 ou 24, **caractérisé en ce que** le composé candidat est choisi dans le groupe de classes de composés constitués par des peptides à liaison de grande affinité, des protéines, des anticorps, des anticalins, des acides nucléiques fonctionnels, des composés naturels et des petites molécules.

26. Procédé selon la revendication 25, **caractérisé en ce que** les acides nucléiques fonctionnels sont choisis dans le groupe qui comprend des aptamères, des aptazymes, des ribozymes, des spiegelmères, des oligonucléotides antisens et le siARN.

27. Utilisation in vitro du facteur de type polypeptide ou d'un fragment de celui-ci tel que défini selon la revendication 1 en tant que marqueur d'activité de PI 3-kinase.

28. Utilisation in vitro d'un acide nucléique tel que défini selon la revendication 6 en tant que marqueur d'activité de PI 3-kinase.

29. Utilisation d'un acide nucléique antisens ou d'une ARN interférant pour la fabrication d'un médicament destiné au traitement d'une maladie, moyennant quoi une telle maladie est un cancer et l'acide nucléique antisens et l'ARN interférant sont dirigés contre un acide nucléique tel que défini selon la revendication 6.

30. Utilisation selon la revendication 29, dans laquelle le cancer est choisi dans le groupe constitué par les carcinomes colorectaux, les gliomes, les cancers de l'endomètre, les adénocarcinomes, les hyperplasies de l'endomètre, le syndrome de Cowden, le cancer des seins et de l'ovaire, le cancer de la prostate, le syndrome de Bannayan-Zonana, le syndrome de Lhermitte-Duclos (LDD), les maladies de l'hamartome-macrocéphale comprenant la maladie de Cowden (CD) et le syndrome de Bannayan-Ruvalcaba-Riley (BRR), les trichilemmomes, les hamartomes gastro-intestinaux, les lipomes, les adénomes de la thyroïde, une maladie fibrokystique du sein, le gangliocytome cérébelleux dysplastique et les malignités du sein et de la tyroïde.
